# EUROPEAN PATENT SPECIFICATION

(11) **EP 4 240 284 B1**
(45) Date of publication and mention of the grant of the patent: **17.07.2024**
(21) Application number: 21819288.8
(22) Date of filing: 05.11.2021
(51) Int. Cl.: A61F 2/24, A61M 25/00, A61F 2/962

(54) **FLEXIBLE CATHETER DEVICES AND METHODS OF MANUFACTURE**
FLEXIBLE KATHETERVORRICHTUNGEN SOWIE VERFAHREN ZUR HERSTELLUNG
DISPOSITIFS CATHÉTERS FLEXIBLES ET PROCÉDÉS DE FABRICATION

(30) Priority: 05.11.2020 US 202063110162 P; 29.01.2021 US 202163143507 P
(43) Date of publication of application: 13.09.2023
(73) Proprietor: Edwards Lifesciences Corporation, Irvine, CA 92614 (US)
(72) Inventor: VALENCIA, Salomon Xavier, Irvine, California 92614 (US); LOPEZ, Jose Luis, Irvine, California 92614 (US); BUZEA, Brad, Irvine, California 92614 (US)
(74) Representative: Eisenführ Speiser
(86) International application number: PCT/US2021/058245
(87) International publication number: WO 2022/098998

(56) References cited:
- WO-A1-2017/165842
- WO-A1-2020/142381
- US-A1- 2004 176 740
- US-A1- 2018 193 042
- US-B1- 10 668 258
- US-B2- 7 438 712

## Description

### CROSS-REFERENCE TO RELATED APPLICATIONS

This application claims benefit of U.S. Provisional Application No. 63/110,162, filed November 5, 2020, and U.S. Provisional Application Nos. 63/143,507, filed January 29, 2021.

### FIELD

The present application is directed to a sheath for use with catheter-based technologies for repairing and/or replacing heart valves, as well as for delivering an implant, such as a prosthetic valve to a heart via the patient's vasculature.

### BACKGROUND

Endovascular delivery catheter assemblies are used to implant prosthetic devices, such as a prosthetic valve, at locations inside the body that are not readily accessible by surgery or where access without invasive surgery is desirable. For example, aortic, mitral, tricuspid, and/or pulmonary prosthetic valves can be delivered to a treatment site using minimally invasive surgical techniques. Percutaneous interventional medical procedures utilize the large blood vessels of the body to reach target destinations rather than surgically opening the target site. There are many types of disease states that can be treated via interventional methods, including coronary blockages, valve replacements (TAVR), and brain aneurysms. These techniques involve using wires, catheters, balloons, electrodes, and other thin devices to travel down the length of the blood vessels from the access site to the target site. The devices have a proximal end, which the clinician controls outside of the body, and a distal end inside the body, which is responsible for treating the disease state. Percutaneous interventional procedures offer several advantages over open surgical techniques. First, they require smaller incision sites, which reduces scarring and bleeding as well as an infection risk. Procedures are also less traumatic to the tissue, so recovery times are reduced. Finally, interventional techniques can usually be performed much faster and with fewer clinicians participating in the procedure, so overall costs are lowered. In some cases, the need for anesthesia is also eliminated, further speeding up the recovery process and reducing risk.

A single procedure typically uses several different guidewires, catheters, and balloons to achieve the desired effect. One at a time, each tool is inserted and then removed from the access site sequentially. For example, a guidewire is used to track to the correct location within the body. Next, a balloon may be used to dilate a section of a narrowed blood vessel. Last, an implant may be delivered to the target site. Because catheters are frequently inserted and removed, introducer sheaths are used to protect the local anatomy and simplify the procedure.

An introducer sheath can be used to safely introduce a delivery apparatus into a patient's vasculature (e.g., the femoral artery). Introducer sheaths are conduits that seal onto the access site blood vessel to reduce bleeding and trauma to the vessel caused by catheters with rough edges. An introducer sheath generally has an elongated sleeve that is inserted into the vasculature and a housing that contains one or more sealing valves that allow a delivery apparatus to be placed in fluid communication with the vasculature with minimal blood loss. Once the introducer sheath is positioned within the vasculature, the shaft of the delivery apparatus is advanced through the sheath and into the vasculature, carrying the prosthetic device. Expandable introducer sheaths, formed of highly elastomeric materials, allow for the dilating of the vessel to be performed by the passing prosthetic device. Expandable introducer sheaths are disclosed in U.S. Patent No. 8,790,387, entitled "Expandable Sheath for Introducing an Endovascular Delivery Device into a Body," U.S. Patent No. 10,639,152, entitled "Expandable Sheath and Methods of Using the Same," U.S. Application No. 14/880,109, entitled "Expandable Sheath," U.S. Application No. 16/407,057, entitled "Expandable Sheath with Elastomeric Cross-Sectional Portions," U.S. Patent No. 10,327,896, entitled "Expandable Sheath with Elastomeric Cross-Sectional Portions," U.S. Application No. 15/997,587, entitled "Expandable Sheath for Introducing an Endovascular Delivery Device into a Body," U.S. Application No. 16/378,417, entitled "Expandable Sheath".

US 2004/176740 A1 discloses a multiple braid exterior tube having a composite structure which includes an inner tubular layer, reinforcing layers and a polymer matrix layer. The exterior tube is formed with polymeric materials and metallic reinforcing braiding configured to provide greater tensile strength and stiffness. The exterior tube is used for a variety of medical devices such as a sheath component for intravascular devices and catheters.

Conventional methods of accessing a vessel, such as a femoral artery, prior to introducing the delivery system include dilating the vessel using multiple dilators or sheaths that progressively increase in diameter. Typically, the introducer is inserted into the sheath during preparation, and both are then inserted into the vessel. Due to the need for a smooth transition from the introducer to the sheath, it is vital that the change in diameter of the introducer occurs distal to the tip of the sheath, such that the tip of the sheath fits snugly around the diameter. During insertion of the sheath and introducer, it is possible that the introducer can move backward within the sheath, eliminating the aforementioned snug fit and creating a lip between the sheath tip and the smaller outer diameter of the introducer. This lip/gap can lead to severe vessel trauma during insertion.

Moreover, some procedures, such as a transseptal approach for mitral valve replacement/repair, require prolonged dilation of incisions in heart tissue and a curving/bending of the sheath to access the treatment site, prolonging procedure time and recovering and increasing the risk of trauma to vessels and heart tissue.

Accordingly, there remains a need for further improvements in expandable introducer sheath for endovascular systems used to implant valves and other prosthetic devices.

### SUMMARY

The claimed invention is defined in independent claim 1 and relates to a flexible delivery catheter. Preferred configurations of the claimed invention are defined in dependent claims 2 to 15. Also described herein are related aspects, examples, embodiments and arrangements useful for understanding the claimed invention.

Aspects of the current disclosure are directed to the flexible delivery catheter comprising an outer jacket, having a proximal end, a distal end, an inner surface and an outer surface, and defining a central lumen extending longitudinally between the proximal end and the distal end; a coil layer comprising a coil winding and having a proximal end, a distal end, an inner surface, and an outer surface, wherein the coil winding extends helically about a longitudinal axis of the delivery catheter and further defines a central lumen between the proximal end and the distal end; a braided layer having a proximal end and a distal end and comprising a plurality of fibers disposed in a braided pattern and defining a central lumen having an inner surface and an outer surface, wherein the coil layer is disposed within the central lumen of the braided layer, and wherein the braided layer is disposed between the outer jacket and the coil layer, wherein the coil layer is wound to resist axial compression and tension applied to the delivery catheter, the coil layer facilitates bending of the delivery catheter in a direction away from the longitudinal axis of the delivery catheter; wherein the coil layer and the braided layer are provided at a distal tip portion of the delivery catheter, wherein the distal tip portion has a first length extending along a length of the delivery catheter from the distal end of the delivery catheter toward the proximal end of the delivery catheter; and wherein the delivery catheter is omnidirectionally flexible and configured to bend in any direction away from the longitudinal axis of the delivery catheter.

In still further aspects, the flexible delivery catheter as disclosed herein comprises a tie layer provided on the inner surface of the coil layer. In yet further aspects, the disclosed herein delivery catheters can further comprise an outer barrier layer provided between the outer surface of the coil layer and an inner barrier layer provided on the inner surface of the coil layer.

In still further aspects, the flexible delivery catheter as disclosed herein can comprise at least one tensile stiffening fiber for limiting bending of the delivery catheter in a direction opposite a circumferential location of the at least one tensile stiffening fiber.

Also disclosed herein is a flexible delivery catheter comprising: an outer jacket, having a proximal end, a distal end, an inner surface, and an outer surface, and defining a central lumen extending longitudinally between the proximal end and the distal end; a braided layer embedded in the outer jacket comprising a plurality of interwoven fibers disposed in a braided pattern forming an elongated tubular structure defining a central lumen extending therethrough, and wherein a braid density of the braided pattern varies along a length of the braided layer; and wherein the delivery catheter is omnidirectionally flexible and configured to bend in any direction away from the longitudinal axis of the delivery catheter.

Also disclosed is a flexible delivery catheter comprising: an outer jacket, having a proximal end, a distal end, an inner surface and an outer surface, and defining a central lumen extending longitudinally between the proximal end and the distal end; and at least one tensile stiffening fiber extending longitudinally along the delivery catheter from the proximal to the distal end of the delivery catheter, the at least one tensile stiffening fiber liming bending of the delivery catheter in a direction opposite the circumferential location of the at least one tensile stiffening fiber.

In still further aspects, disclosed is a flexible delivery catheter comprising: an outer jacket, having a proximal end, a distal end, an inner surface, and an outer surface, and defining a central lumen extending longitudinally between the proximal end and the distal end; a braided layer comprising a plurality of fibers disposed in a braided pattern, wherein the braided layer is positioned within the central lumen, a hypotube having a proximal end, a distal end, an inner surface, an outer surface, and a plurality of cut-outs which extend between the inner surface and the outer surface of the hypotube such that the hypotube can bend omnidirectionally, and wherein the hypotube is provided at a distal tip portion of the delivery catheter, the distal tip portion extending along a length of the delivery catheter from a distal end of the delivery catheter toward the proximal end of the delivery catheter.

Also disclosed are methods for making a flexible delivery catheter comprising: forming an outer jacket, having a proximal end, a distal end, an inner surface, and an outer surface, and defining a central lumen extending longitudinally between the proximal end and the distal end; forming a coil layer comprising a coil winding and having a proximal end, a distal end, an inner surface, and an outer surface, wherein the coil winding extends helically about a longitudinal axis and defines a central lumen between the proximal end and the distal end; forming a braided layer having a proximal end and a distal end and comprising a plurality of wires disposed in a braided pattern and defining a central lumen having an inner surface and an outer surface, disposing the coil layer within the central lumen of the braided layer, disposing the braided layer between the outer jacket and the coil layer; and coupling the outer jacket, the coil layer and the braided layer together.

Also disclosed are methods for making a flexible delivery catheter comprising forming an outer jacket, having a proximal end, a distal end, an inner surface, and an outer surface, and defining a central lumen extending longitudinally between the proximal end and the distal end; forming a braided layer comprising a plurality of wires disposed in a braided pattern and defining a central lumen; and forming a hypotube having a proximal end, a distal end, an inner surface, an outer surface, and a plurality of cut-outs which extend between the inner surface and the outer surface of the hypotube such that the hypotube can bend omnidirectionally, wherein the hypotube is provided at a distal tip portion of the delivery catheter, the distal tip portion extending along a length of the delivery catheter from a distal end of the delivery catheter toward the proximal end of the delivery catheter.

In still further aspects, also disclosed are methods of making a flexible delivery catheter comprising forming an outer jacket, having a proximal end, a distal end, an inner surface, and an outer surface, and defining a central lumen extending longitudinally between the proximal end and the distal end; braiding a plurality of interwoven fibers in a braided pattern to form a braided layer, embedding the braided layer in the outer jacket forming an elongated tubular structure defining a central lumen extending therethrough, and wherein a braid density of the braided pattern varies along a length of the braided layer.

Still further disclosed are also methods of making a flexible delivery catheter comprising forming an outer jacket, having a proximal end, a distal end, an inner surface, and an outer surface, and defining a central lumen extending longitudinally between the proximal end and the distal end; forming a braided layer embedded in the outer jacket comprising a plurality of interwoven fibers disposed in a braided pattern forming an elongated tubular structure defining a central lumen extending therethrough; coupling at least one tensile stiffening fiber to the outer jacket, and wherein the tensile stiffening fiber extends longitudinally along the delivery catheter from the proximal to the distal end of the delivery catheter, the at least one tensile stiffening fiber limiting bending of the delivery catheter in a direction opposite the circumferential location of the at least one tensile stiffening fiber.

Yet still further, disclosed are methods of deploying a prosthetic into a patient comprising: providing a delivery system comprising a flexible catheter having a proximal end and a distal end, wherein the outer jacket having an elasticity that varies (longitudinally) along a length of the outer jacket; advancing the catheter into a vessel of a patient, and advancing the prosthetic from the proximal end of the catheter to the distal end of the catheter and into the patient.

Also disclosed is a flexible delivery catheter having a proximal end and a distal end and comprising: an outer jacket, having a proximal end, a distal end, an inner surface and an outer surface, and defining a central lumen extending longitudinally between the proximal end and the distal end; a stent layer having a proximal end, a distal end, an inner surface, and an outer surface; an inner barrier layer disposed abut the inner surface of the stent layer; and an outer barrier layer disposed abut the outer surface of the stent layer; wherein the stent layer is disposed within the central lumen; wherein the stent layer is configured to resist axial compression and tension applied to the delivery catheter, the stent layer is configured to facilitate bending of the delivery catheter in a direction away from the longitudinal axis of the delivery catheter, and wherein the stent layer is provided at a distal tip portion of the delivery catheter, the distal tip portion has a length and extends along a length of the delivery catheter from the distal end of the delivery catheter toward the proximal end of the delivery catheter.

In still further aspects, disclosed is a flexible delivery catheter comprising: an outer jacket, having a proximal end, a distal end, an inner surface and an outer surface, and defining a central lumen extending longitudinally between the proximal end and the distal end; a coil layer comprising a coil winding and having a proximal end, a distal end, an inner surface, and an outer surface, wherein the coil winding extends helically about a longitudinal axis of the delivery catheter, a first barrier layer disposed at the inner surface of the coil layer; a second barrier layer disposed at the outer surface of the coil layer; and wherein the coil layer is wound to resist axial compression and tension applied to the delivery catheter, the coil layer facilitates bending of the delivery catheter in a direction away from the longitudinal axis of the delivery catheter, and wherein the coil layer is provided at a distal tip portion of the delivery catheter, wherein the distal tip portion has a first length extending along a length of the delivery catheter from the distal end of the delivery catheter toward the proximal end of the delivery catheter.

While in still further aspects, disclosed is a flexible delivery catheter comprising: an outer jacket, having a proximal end, a distal end, an inner surface and an outer surface, and defining a central lumen extending longitudinally between the proximal end and the distal end; a hypotube having a proximal end, a distal end, an inner surface, an outer surface, and a plurality of cut-outs which extend between the inner surface and the outer surface of the hypotube such that the hypotube can bend omnidirectionally, an inner barrier coating layer disposed at at least a portion of the inner surface of the hypotube; an outer barrier coating layer disposed at at least a portion of on the outer surface of the hypotube, and wherein the hypotube is provided at a distal tip portion of the delivery catheter, the distal tip portion extending along a length of the delivery catheter from a distal end of the delivery catheter toward the proximal end of the delivery catheter.

In still further aspects disclosed is a method of making a flexible delivery catheter comprising: forming an outer jacket, having a proximal end, a distal end, an inner surface, and an outer surface, and defining a central lumen extending longitudinally between the proximal end and the distal end; forming a stent layer having a proximal end, a distal end, an inner surface, and an outer surface defining a central lumen between the proximal end and the distal end; disposing an inner barrier layer at the inner surface of the coil layer; disposing an outer barrier layer at the outer surface of the coil layer; disposing the coil layer and the inner barrier layer and the outer barrier layer within the central lumen of the outer jacket; and wherein the delivery catheter is omnidirectionally flexible and configured to bend in any direction away from the longitudinal axis of the delivery catheter.

### DESCRIPTION OF DRAWINGS

FIG. 1 is a side view of the heart valve delivery system delivering a heart valve to a native valve site according to one aspect of the present disclosure.
FIG. 2 is a perspective view of a delivery catheter (delivery sheath) of FIG. 1B
FIG. 3 is a partial side cross section view (not to scale) of an example flexible catheter, including a coil layer and a braided layer extending along the entirety (or a portion) of the length of the catheter.
FIG. 4 is a partial side cross section view (not to scale) of an example flexible catheter, including a coil layer and a braided layer.
FIG. 5 is a partial side cross section view (not to scale) of an example flexible catheter, including a coil layer, a braided layer, and a PET layer surrounding the coil layer.
FIG. 6 is a partial side cross section view (not to scale) of an example flexible catheter having a variable density braided layer.
FIG. 7 is a partial side cross section view (not to scale) of an example flexible catheter having a braided layer.
FIG. 8 is a partial side cross section view (not to scale) of an example flexible catheter that comprises a hypotube.
FIG. 9 is a partial side cross section view (not to scale) of an example flexible catheter that comprises a hypotube.
FIG. 10 is a side view of the example hypotube of FIG. 8 or FIG. 9.
FIG. 11 is a finite element analysis of the stress experience by the example hypotube of FIG. 10 during bending.
FIG. 12 is a flattened view of the hypotube shown in FIG. 8 or FIG. 9.
FIG. 13 is a side and end view of an example hypotube.
FIG. 14 is a partial side cross section view (not to scale) of an example flexible catheter in one aspect.
FIG. 15 is a partial side cross section view (not to scale) of an example flexible catheter in one aspect.

### DETAILED DESCRIPTION

Other examples, features, aspects, embodiments, and advantages will become apparent to those skilled in the art from the following description. As will be realized, the device and/or methods are capable of other different and obvious aspects, all without departing from the spirit of the inventive concepts. Accordingly, the drawings and descriptions should be regarded as illustrative in nature and not restrictive.

For purposes of this description, certain aspects, advantages, and novel features of the aspects of this disclosure are described herein. The described methods, systems, and apparatus should not be construed as limiting in any way. Instead, the present disclosure is directed toward all novel and nonobvious features and aspects of the various disclosed aspects, alone and in various combinations and sub-combinations with one another. The disclosed methods, systems, and apparatus are not limited to any specific aspect, feature, or a combination thereof, nor do the disclosed methods, systems, and apparatus require that any one or more specific advantages be present or problems be solved.

Features, integers, characteristics, compounds, chemical moieties, or groups described in conjunction with a particular aspect, embodiment, or example of the disclosure are to be understood to be applicable to any other aspect, embodiment, or example described herein unless incompatible therewith. All of the features disclosed in this specification (including any accompanying claims, abstract, and drawings) and/or all of the steps of any method or process so disclosed may be combined in any combination, except combinations where at least some of such features and/or steps are mutually exclusive. The disclosure is not restricted to the details of any foregoing aspects. The disclosure extends to any novel one, or any novel combination, of the features disclosed in this specification (including any accompanying claims, abstract, and drawings), or to any novel one, or any novel combination, of the steps of any method or process so disclosed.

Throughout the disclosure 1 inch corresponds to 25.4 mm

As used in the specification and the appended claims, the singular forms "a," "an," and "the" include plural referents unless the context clearly dictates otherwise. Thus, for example, a reference to a "segment" includes aspects having two or more such materials unless the context clearly indicates otherwise.

Ranges may be expressed herein as from "about" one particular value and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint and independently of the other endpoint. Unless stated otherwise, the term "about" means within 5% (e.g., within 2% or 1%) of the particular value modified by the term "about."

Throughout this disclosure, various aspects of the disclosure can be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6, etc., as well as individual numbers within that range, for example, 1, 2, 2.7, 3, 4, 5, 5.3, 6 and any whole and partial increments therebetween. This applies regardless of the breadth of the range.

"Optional" or "optionally" means that the subsequently described event or circumstance may or may not occur and that the description includes instances where said event or circumstance occurs and instances where it does not.

The terms "proximal" and "distal" as used herein refer to regions of a sheath, catheter, or delivery assembly. "Proximal" means that region closest to handle of the device, while "distal" means the region farthest away from the handle of the device.

"Axially" or "axial" as used herein refers to a direction along the longitudinal axis of the sheath.

Throughout the description and claims of this specification, the word "comprise" and variations of the word, such as "comprising" and "comprises," means "including but not limited to," and is not intended to exclude, for example, other additives, components, integers or steps. As used in the specification and in the claims, the term "comprising" can include the aspects "consisting of" and "consisting essentially of." Additionally, the term "includes" means "comprises."

"Exemplary" means "an example of" and is not intended to convey an indication of a preferred or ideal aspect. "Such as" is not used in a restrictive sense but for explanatory purposes.

It will be understood that when an element is referred to as being "connected" or "coupled" to another element, it can be directly connected or coupled to the other element, or intervening elements may be present. In contrast, when an element is referred to as being "directly connected" or "directly coupled" to another element, there are no intervening elements present. Other words used to describe the relationship between elements or layers should be interpreted in a like fashion (e.g., "between" versus "directly between," "adjacent" versus "directly adjacent," "on" versus "directly on").

As used herein, the term "and/or" includes any and all combinations of one or more of the associated listed items.

It will be understood that, although the terms "first," "second," etc., may be used herein to describe various elements, components, regions, layers, and/or sections. These elements, components, regions, layers, and/or sections should not be limited by these terms. These terms are only used to distinguish one element, component, region, layer, or section from another element, component, region, layer, or a section. Thus, a first element, component, region, layer, or section discussed below could be termed a second element, component, region, layer, or section without departing from the teachings of the example aspect.

Spatially relative terms, such as "beneath," "below," "lower," "above," "upper," and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "below" or "beneath" other elements or features would then be oriented "above" the other elements or features. Thus, the term "below" can encompass both an orientation of above and below. The device may be otherwise oriented (rotated 90 degrees or at other orientations), and the spatially relative descriptors used herein interpreted accordingly.

As used herein, the term "substantially," in, for example, the context "substantially identical" or "substantially similar" refers to a method or a system, or a component that is at least about 90%, at least about 91%, at least about 92%, at least about 93%, at least about 94%, at least about 95%, at least about 96%, at least about 97%, at least about 98%, at least about 99%, or about 100% by similar to the method, system, or the component it is compared to.

The term "tube" or "tubular" as used herein is not meant to limit shapes to circular cross-sections. Instead, tube or tubular can refer to any elongate structure with a closed-cross section and lumen extending axially therethrough. A tube may also have some selectively located slits or openings therein - although it still will provide enough of a closed structure to contain other components within its lumen(s).

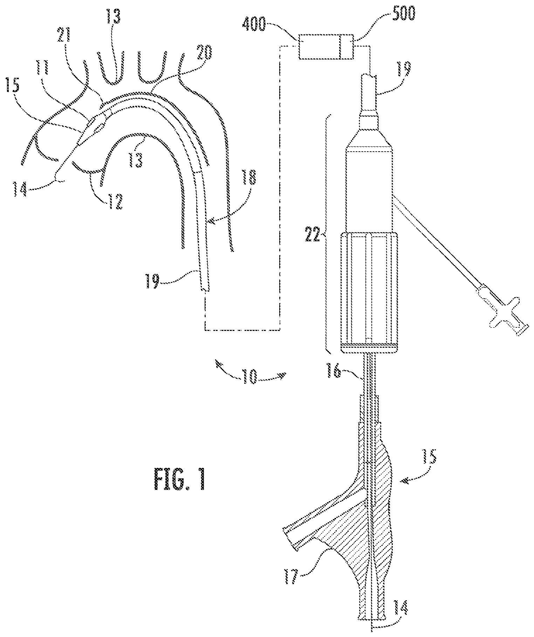

An exemplary delivery system is shown in FIG. 1. FIG. 1, for purposes of illustration, shows an aspect of a heart valve delivery system 10 for delivering a prosthetic valve 11 to a diseased aortic valve 12 of a human heart is shown. The delivery system is well-suited for delivering the prosthetic valve 11 through a patient's vasculature and over an aortic arch 13 to a location adjacent to the diseased valve 12.

The delivery system 10 generally includes a guidewire 14 and a balloon catheter 15 configured for advancement over the guidewire 14. The prosthetic valve 11 is provided along the distal end portion of the balloon catheter. The balloon catheter 15 includes a tubular section 16 and a handle/support 17 at a proximal end of the tubular section 16. The tubular section 16 of the balloon catheter 15 is received within a delivery sleeve assembly 18. The delivery sleeve assembly generally comprises an elongated polymeric sleeve 19 as disclosed in detail herein, a steerable section 20, and a shroud section 21. A proximal end of the delivery sleeve assembly 18 is mounted to a handle 22. The delivery system 10 passes through an introducer sheath assembly 400 and a loader assembly 500, both of which will be described in more detail below, to enter the body vessel and deliver the valve 11.

The following figures demonstrate described herein aspects in more detail. Additional exemplary aspects can also be found in U.S. Patent No. 7,780,723, U.S. Patent No. 8,382,826, U.S. Patent No. 9,028,545, U.S. Patent No. 9,907,651, and U.S. Patent No. 10,478,294.

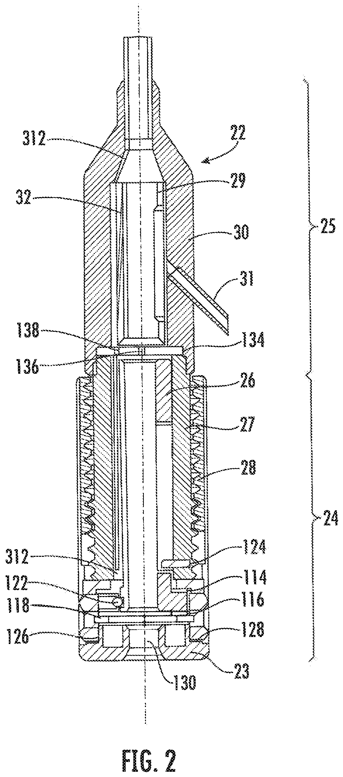

With reference to FIG. 2, the handle 22 at the proximal end of the delivery sleeve assembly 18 generally includes an end cap 23, an adjustable portion 24, and a hemostasis portion 25. The adjustable portion 24 includes a first core member 26, a partially threaded member 27 around the first core member 26, and a rotator handle 28 around the partially threaded member 27. The hemostasis portion 25 includes a second core member 29 and a hub 30 around the second core member 29. A hemostasis tube 31 extends outwards from the hub 30. A guide tube 32 is placed within the handle 22, as will be described in greater detail below.

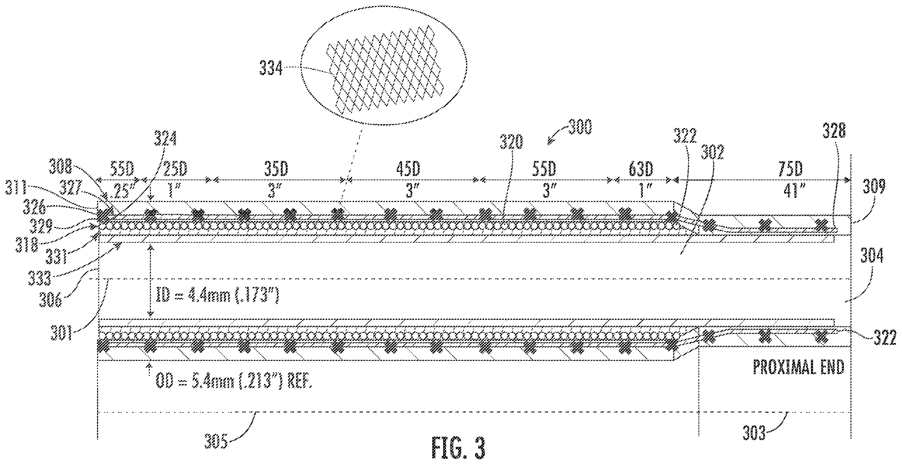

FIG. 3 shows a flexible introducer catheter, i.e., delivery catheter 300. The delivery catheter 300 can provide an omnidirectional flexible catheter that is bendable in any direction away from a longitudinal axis 301 of the delivery catheter 300 while retaining compressive and tensile stiffness. As such, the flexible delivery catheter 300 conforms to a patient's vascular structure during placement and delivering a medical device (e.g., prosthetic heart valve) while limiting axial deformation of the delivery catheter 300 that can damage a patient's vascular structure. As will be described in more detail below, the delivery catheter 300 includes a coil layer 318, which provides compressive stiffness to the catheter 300, a braided layer 326, which provides structural rigidity, a tensile stiffening fiber 327, which provides tensile stiffness, and an outer jacket 308 having various elasticity characteristics along its axial length. The delivery catheter 300 defines an elongated tubular structure with a central lumen 302 extending longitudinally between the proximal end 304 and the distal end 306. The central lumen 302 (and in each aspect described below) is configured for passage of medical devices such as prosthetic heart valves therethrough. The delivery catheter 300 includes an elongated body portion 303 and a distal tip portion 305. The elongated body portion 303 extends from the distal tip portion 305 (at the distal end 306 of the delivery catheter 300) to the proximal end 304 of the delivery catheter 300. The elongated body portion 303 provides a section of the catheter 300 that is rigid enough for effective manipulation by a physician (e.g., advancement through the patient's vasculature). The distal tip portion 305 has a less rigid structure than the elongated body portion 303, which is suitable for conforming to the vascular structure of a patient during a delivery procedure, as described above.

As illustrated in FIG. 3, the coil layer 318 and the braided layer 326 are provided at the distal tip portion 305 of the delivery catheter 300. The coil layer 318 is disposed within the inner lumen of the braided layer 326. The braided layer 326 is disposed between the outer jacket 308 and the coil layer 318. The coil layer 318 and the braided layer 326 are provided at the distal tip portion 305 of the delivery catheter 300. As such, the coil layer 318 is wound to resist axial compression and tension applied to the delivery catheter 300, the coil layer 318 facilitates bending of the delivery catheter 300 at the distal tip portion 305 in a direction away from the longitudinal axis 301 of the delivery catheter 300.

As illustrated in FIG. 3, the distal tip portion 305 of the delivery catheter 300 has an outer diameter that is greater than an outer diameter of the elongated body portion 303, whereas the inner diameter of the delivery catheter 300, along both the elongated body portion 303 and the distal tip portion 305, remains constant. The outer surface of the distal tip portion 305 adjacent the elongated body portion 303 includes a tapered surface extending toward the outer surface of the elongated body portion 303. This decreasing taper is provided between the larger outer diameter distal tip portion 305 and the reduced diameter elongated body portion 303.

In the example shown in FIG. 3, the outer diameter of the elongated body portion 303 is about 5.4 mm. But in other examples, the outer diameter of the elongated body portion 303 ranges between about 4.5 mm and about 6.0 mm or between about 5.0 mm and about 6.0 mm. In the example shown in FIG. 3, the outer diameter of the distal tip portion 305 is about 5.59 mm. In other examples, the outer diameter of the distal tip portion 305 ranges between about 4.5 mm and about 6.0 mm, or between about 5.0 mm and about 6.0 mm. In the example shown in FIG. 3, the inner diameter of the central lumen 302 of the delivery catheter 300 is about 4.4 mm. In other examples, the inner diameter of the central lumen 302 of the delivery catheter 300 ranges between 3.5 mm and about 5.0 mm or between 4.0 mm and about 4.5 mm.

The distal tip portion 305 extends along a length of the delivery catheter 300 from a distal end 306 of the delivery catheter 300 toward the proximal end 304 of the delivery catheter 300. In the example shown in FIG. 3, the length of the distal tip portion 305, which is measured in a direction along the longitudinal axis of the delivery catheter 300, is about 285.75 mm (11.25 inches). In some examples, the distal tip portion 305 has a length less than 508 mm (20 inches), less than 381 mm (15 inches), between 127 mm (5 inches) and 381 mm (15 inches), or between 254 mm (10 inches) and 381 mm (15 inches). In the example shown in FIG. 3, the length of the elongated body portion 303 is about 1041.4 mm (41 inches). In some examples, the length of the elongated body portion 303 ranges between about 762 mm (30 inches) to about 1270 mm (50 inches) or between about 889 mm (35 inches) to about 1143 mm (45 inches).

As described above, the catheter 300 includes a coil layer 318. The coil layer 318 is wound to resist axial compression applied to the delivery catheter 300 while facilitating bending of the delivery catheter 300 in a direction away from the longitudinal axis of the delivery catheter 300. The coil layer 318 includes a coil winding 320, which provides compressive stiffness to the catheter 300 during axial compressive loads. The coil layer 318 has a proximal end 322, a distal end 324, an inner surface, and an outer surface. The coil winding 320 extends helically about a longitudinal axis and defines a central lumen between the proximal end 322 and the distal end 324. In the example shown in FIG. 3, the coil layer 318 has a plurality of tightly wound turns of the coil winding 320. In an example coil layer 318, a gap/spacing is provided between adjacent turns of the coil winding 320. In another example, the coil layer 318 is tightly wound such that the adjacent turns of the coil winding 320 contact, i.e., an outer surface of the adjacent turns of the coiled winding 320 contact along a circumferential length of the coiled winding 320. This allows for some axial compression in certain applications that require partial axial compression of the catheter. Adjacent at the ends of the coil layer 318 are welded together to control axial expansion and placement of the coil 318 during manufacturing. In the example shown in FIG. 3, the coil layer 318 has a length that is less than a length of the distal tip portion 305. But in other examples, a length of the coil layer 318 is greater than a length of the distal tip portion 305 such that the coil layer 318 extends into the elongated body portion 303 of the delivery catheter 300.

In the example shown in FIG. 3, the coil winding 320 has a diameter ranging between about 0.003 inches and about 0.010 inches, and between about 0.004 inches and about 0.008 inches. In one example, coil winding 320 has a diameter of about 0.004 inches. In other examples, coil winding 320 has a diameter of about 0.008 inches.

As described above, the coil windings 320 have a constant pitch along the axial length of the coil layer 318. But in other implementations, the coil windings 320 have a varying pitch along an entire length of the coil winding 320/coil layer 318. For example, a pitch of the coil winding 320 at a proximal end of the distal tip portion 305, e.g., along the tapered portion of the distal tip portion 305, can be less than a pitch of the coil winding 320 at the distal end 306 of the distal tip portion 305. In the example shown in FIG. 3 the coil layer 318 has about 55 turns per inch, but in other examples, the coil has between about 50 and 120 turns per inch, in implementations having 0.008 inch diameter coil wire.

The coil layer 318 can be composed of at least one a polymer, a metal, or a composite. For example, the coil layer 318 can be composed of a stainless steel wire. The coil winding 320 has a curvilinear shape in the cross section, but the coil winding 320 can have either a rectilinear or curvilinear shape in the cross section.

As described above, the catheter 300 includes a braided layer 326, which provides structural support for catheter 300. The braided layer 326 has a proximal end and a distal end. The braided layer 326 includes a plurality of fibers 334 interwoven into a braided pattern and defines a central lumen, an inner surface, and an outer surface. In the example shown in FIG. 3, the braided layer 326 extends along the entire length of the elongated body portion 303 and the distal tip portion 305 of the catheter 300. In another example, the braided layer 326 extends along an entire length of the distal tip portion 305. The braided layer 326 extends along at least a portion of the entire length of the elongated body portion 303 of the delivery catheter 300. In the example shown in FIG. 3 the braided layer 326 extends along the entire length of the catheter 300. As such, braided layer 326 has a length that is greater than an overall length of the coil layer 318. But, in some implementations, the braided layer 326 and the coil layer 318 are about the same length along the axis of the catheter 300.

As described above, the braided layer 326 is composed of a plurality of interwoven fibers 334. Each of the fibers 334 has a curvilinear cross section, although, in other examples, the fibers 334 have a rectilinear cross section. The fibers 334 are composed of stainless steel, but in other examples, the fibers 334 are composed of at least one of a polyester, a polymer, a metal, a composite, or any other material suitable for composing a stiffening braided layer 326 in a catheter 300. Each of the plurality of interwoven fibers 334 has a diameter of about 0.004 inches. In other examples, each of the plurality of interwoven fibers 334 has a diameter ranging between about 0.002 inches and about 0.006 inches or between about 0.003 inches and about 0.005 inches. The interwoven fibers 334 can be oriented in a single strand braid pattern or in a double strand braid pattern where each interweave includes a plurality of parallel fibers 334 for each weave strand. The plurality of interwoven fibers 334 is composed of 16 individual fibers 334. But in other examples, the plurality of interwoven fibers 334 is composed of about 10 to about 20 individual fibers 334. The braided layer 326 has a braid density, which allows for omnidirectional bending with respect to the central axis of the catheter 300. As the braid density increases, the braided layer 326 becomes more resistant to bending. In the example shown in FIG. 3, the braid density of the braided layer 326 is about 25 picks per inch. But in other examples, the braided layer 326 has a braid density between about 20 to about 30 picks per inch.

As described above, the catheter 300, as shown in FIG. 3 includes at least one tensile stiffening fiber 327, having a proximal end 328 and a distal end 329. The tensile stiffening fiber 327 limits tensile deformation along the axial length of the catheter 300 such that the bending of the catheter 300 is limited at a location/along the side corresponding to the circumferential location of the tensile stiffening fiber 327. For example, if the tensile stiffening fiber is located at a 90-degree position (e.g., 3 o'clock) around the circumference of the delivery catheter 300, the delivery catheter 300 will resist bending at the circumferentially 90-degree position (e.g., 3 o'clock). Generally, the location of the tensile stiffening fiber 327 determines the neutral bending axis of the delivery catheter 300. That is, the stiffness and relative location of the tensile stiffening fiber 327 (or location of multiple fibers with respect to each other) defines the line or plane along the delivery catheter 300 at which no extension or compression occurs when the catheter is bent. For example, when the delivery catheter 300 includes a single tensile stiffening fiber 327, the neutral axis of the delivery catheter is defined by the axis of the tensile stiffening fiber 327.

The tensile stiffening fiber 327 extends along at least a portion of the entire length of the elongated body portion 303 of the delivery catheter 300. In the example shown in FIG. 3 the least one tensile stiffening fiber 327 extends along an entire length the elongated body portion 303 of the delivery catheter 300. But, in other examples, a length of the at least one tensile stiffening fiber 327 corresponds to a length of the distal tip portion 305 or is greater than the length of the distal tip portion 305.

The tensile stiffening fiber 327 is coupled to the catheter 300 by weaving/intertwining the tensile stiffening fiber 327 within the fibers/braid pattern of the braided layer 326. The tensile stiffening fiber 327 is further secured to the braided layer 326 when the outer jacket 308 is reflowed into the braided layer 326 during manufacturing. As will be described in more detail below, the distal end of the catheter 300 includes materials of varying durometer along the length of the outer jacket 308. A length/segment of the delivery catheter 300 proximate the distal end 306 of the distal tip portion 305 comprises a material having a higher durometer than the next adjacent segment of the delivery catheter 300. Reflow of the tensile stiffening fiber 327 with the higher durometer material at the distal end 306 of the distal tip portion 305 results in higher friction between the tensile stiffening fiber 327 and the higher durometer material of the outer jacket 308, thereby preventing axial slippage/movement of the tensile stiffening fiber 327 at the distal end of the catheter.

Similarly, the proximal end of the delivery catheter 300 includes a length/segment of a material having a higher durometer than the next adjacent segment of the delivery catheter 300. Accordingly, reflow of the tensile stiffening fiber 327 with the higher durometer material at the proximal end 304 of the delivery catheter 300 prevents slippage/movement of the tensile stiffening fiber 327 with respect to the end of the delivery catheter 300. As such, the tensile stiffening fiber 327 is secured both along the length of the fiber and at its proximal and distal ends, i.e., the tensile stiffening fiber 327 is fixedly coupled to the proximal and distal ends of the outer jacket 308/braided layer 326 and along the along the braided layer 326.

As shown in FIG. 3, the tensile stiffening fiber 327 is woven into the braided layer 326. In the example shown in FIG. 3, the at least one tensile stiffening fiber 327 is woven into the braided layer 326 along an entire length of the braided layer 326. But, in other examples, it is contemplated that the tensile stiffening fiber 327 is woven into a portion of the length of the braided layer 326. For example, the at least one tensile stiffening fiber 327 may not be woven into a first portion of the braided layer 326 extending along the distal tip portion 305 of the delivery catheter 300 but will be woven into a second portion of the braided layer 326 extending along an elongated body portion 303 of the delivery catheter 300 between the distal tip portion 305 and the proximal end 304 of the delivery catheter 300.

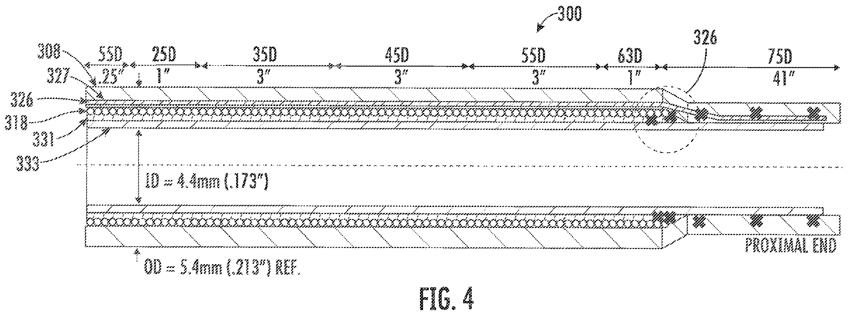

The example is shown in FIG. 3 and FIG. 4 include Technora^{®} Kevlar. But other examples include a tensile stiffening fiber 327 composed of at least one of a polymer, a metal, a composite including a polyamide type polymer, Kevlar or Liquid Crystal Polymer (LCP).

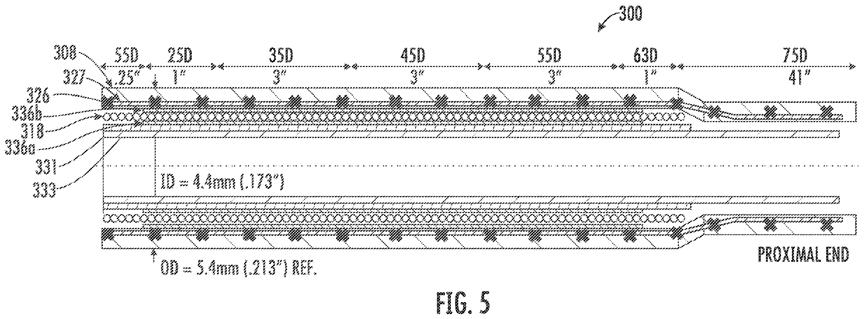

The example, shown in FIG. 3 and FIG. 5, includes four tensile stiffening fibers 327 spaced around the circumference of the catheter 300 by 90 degrees. But other examples include six or less, four or less, two or less, or one individual tensile stiffening fiber 327. In examples having a plurality of tensile stiffening fibers 327, the plurality of individual tensile stiffening fibers 327 can be equally spaced around the circumference of the delivery catheter 300 (e.g., every 60 degrees, 90 degrees, 120 degrees, 180 degrees), or irregularly spaced around the circumference of the delivery catheter 300. An example of having one individual tensile stiffening fiber 327 is also disclosed.

As described above, the catheter 300 includes an outer jacket 308, having a proximal end 309 and a distal end 311, which forms the outermost layer of catheter 300. The outer jacket 308 is composed of an elastic material. The elasticity of the outer jacket 308 varies (longitudinally) along a length of the outer jacket 308. In the example shown in FIG. 3 and FIG. 5, the elasticity of the outer jacket 308 increases between the proximal and distal end 311 of the outer jacket 308 such that the distal end 306 (e.g., distal tip portion 305) of the delivery catheter 300 more easily bends to conform to the aortic geometry of a patient. The durometer of the outer jacket 308 decreases between the proximal end 304 and the distal end 306 of the catheter 300. However, as described above, the distal-most and proximal-most segments of the delivery catheter 300 is constructed from a material having a higher durometer (and lower elasticity) than the next adjacent segment to facilitate coupling of the tensile stiffening element 327 to the proximal and distal ends of the delivery catheter 300. The outer jacket 308 includes various longitudinal segments, which are constructed from materials having varying durometer. The materials of the various longitudinal segments are reflowed together to form a single outer jacket 308. In the example, the delivery catheter 300 as illustrated in FIGS. 3 and 5, the delivery catheter 300 is divided into various length segments. The distal tip portion 305 includes seven segments of various lengths and durometer material.

For example, the first/distal-most segment is composed of a material having a durometer of 55D and a length of about 0.25 inches. The second segment is composed of a material having a durometer of 25D and a length of about 1 inch. The third segment is composed of a material having a durometer of 35D and a length of about 3 inches. The fourth segment is composed of a material having a durometer of 45D and a length of about 3 inches. The fifth segment is composed of a material having a durometer of 63D and a length of about 1 inch. The sixth/proximal-most segment (along the elongated body portion 303 of the delivery catheter 300) is composed of a material having a durometer of 75D and a length of about 41 inches. It is contemplated that the number, length, and durometer of the various segments may vary from those illustrated and described above. As mentioned above, the distal-most segment (referred to also as a coupling segment) and the proximal-most segment (on the elongated body portion 303) have a lower elasticity and higher durometer than the next adjacent segment of the outer jacket 308 to help secure the tensile stiffening fiber 327 to the outer jacket 308 post-reflow procedure.

In the example shown in FIG. 3 the outer jacket 308 is composed of PEBAX@. But, in other examples, at least a portion of the outer jacket 308 comprises another polyether block amide, another elastomer, a polyamide (e.g., Vestamid^{®}), or any other material suitable for forming the outermost layer of a flexible catheter.

In addition to the components described above, the catheter 300 in the examples shown in FIGS. 3-5 include a tie layer 331. The tie layer 331 is provided on an inner surface of the coil layer 318 and creates a surface for improved adhesion between the coil layer 318 and the liner 333, which is described below. The tie layer 331 bonds the liner 333 to the outer jacket 308. The tie layer 331 is reflowed and/or bonded with the outer jacket 308. The tie layer does not bond to the coil layer 318, instead, it encapsulates the coil layer 318, preventing material from entering between the coil windings. As illustrated in FIG. 3, the tie layer 331 extends along the entire axial length of the inner surface of the coil layer 318. In other examples, as shown in FIG. 5, the tie layer 331 extends along a portion of the axial length of the coil layer 318. The tie layer 331 has a thickness of about 0.003 inches, but in other examples, the tie layer 331 has a thickness ranging between about 0.002 inches and about 0.005 inches. The tie layer 331 comprises a polyether block amide material such as PEBAX@, and/or a polyamide (e.g., Vestamid^{®}), polyamide 12 (e.g., Rilsamid^{®}), or any other suitable material.

As described above, the catheter 300 includes a liner 333 provided on an inner surface of the tie layer 331. An inner surface of the liner 333 defines an inner lumen of the delivery catheter 300. The liner 333 extends along an entire length of the inner surface of the tie layer 331 and/or the inner lumen of the delivery catheter 300. In some examples, liner 333 has a length that is greater than a length of the distal tip portion 305. In some examples, the liner 333 extends along at a portion of the entire length of the elongated body portion 303 of the delivery catheter 300 or the entire length of the elongated body portion 303 of the delivery catheter 300. The liner 333 in the example shown in FIG. 3 has a thickness of about 0.002 inches. But in other examples, the liner 333 has a thickness ranging between 0.001 inches and about 0.004 inches or between 0.0015 inches and about 0.003 inches. The liner 333 is composed of polytetrafluoroethylene (PTFE), Polyamide 12 materials, polyether block amide (PEBA), fluorinated ethylene propylene (FEP), or any other polymer suitable for forming an innermost layer of a flexible delivery catheter 300.

The example shown in FIG. 5 includes a barrier layers 336a-b formed from polyethylene terephthalate (PET). In other examples, the barrier layers are formed from other polymers. The flexible delivery catheter 300 in the example shown in FIG. 5 the PET layer forms an outer barrier layer 336b between an outer surface 317 of the coil layer 318 and the inner surface of the outer jacket 308/braided layer 326. An inner barrier layer 336a is provided between the inner surface of the coil layer 318 and the tie layer 331. The inner and outer barrier layers 336a, 336b extend along a majority of the length of the coil layer 318. The inner and outer barrier layers 336a, 336b are not provided along a distal length of the coil layer 318, e.g., a length extending proximally from the distal end 324 of the coil layer 318. Accordingly, the distal end 324 of the coil layer 318 is fixedly coupled at the distal end 306 of the delivery catheter 300 when the outer jacket 308 bonded (e.g., reflowed) to the tie layer 331.

Similarly, the inner and outer barrier layers 336a, 336b are not provided along a length of the coil layer 318 extending distally from the proximal end 322 of the coil layer 318 such that the proximal end 322 of the coil layer 318 is fixedly attached to the outer jacket 308. Like the distal end 324, the proximal end 322 of the coil layer 318 is fixedly coupled to the outer jacket 308 when the outer jacket 308 is bonded (e.g., reflowed) to the tie layer 331. In the example shown in FIG. 5, the inner and outer barrier layers have a thickness of about 0.005 inches. But, in other examples, the inner and outer barrier layers have a thickness ranging between about 0.0003 inches and about 0.0007 inches or between about 0.0004 inches and about 0.0006 inches.

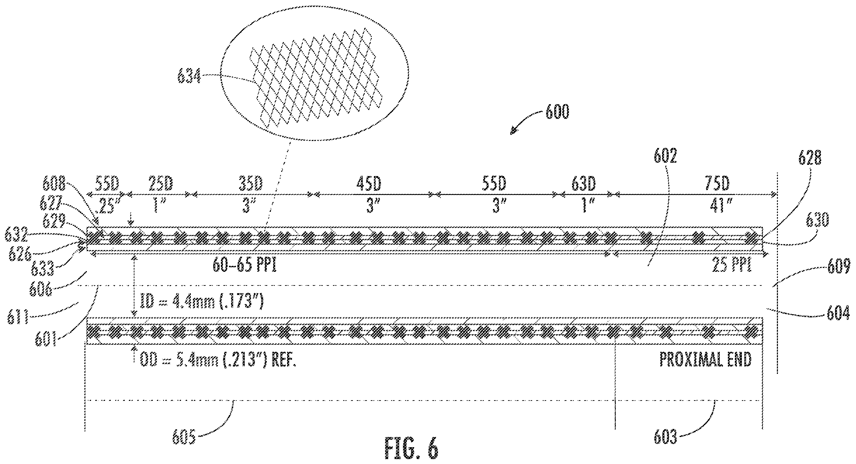

FIG 6 shows another aspect of the flexible catheter 600 having a variable braid density braided layer 626. The catheter 600 includes similar structures and features to catheter 300 described above, the differences will be discussed in more detail below, like element numbers will be used to identify like elements. The catheter 600 has a proximal end 604 and a distal end 606, an inner surface 607, and an outer surface 610 and includes an outer jacket 608, having a proximal end 609, a distal end 611. The delivery catheter 600 defines a central lumen 602 extending longitudinally between the proximal end 604 and the distal end 606. The braided layer 626 is embedded in the outer jacket 608 to provide structural rigidity. The braided layer 626 has a plurality of interwoven fibers disposed in a braided pattern forming an elongated tubular structure defining a central lumen 627 extending therethrough. A braid density of the braided pattern varies along a length of the braided layer 626, as described above. The delivery catheter 600 is omnidirectionally flexible and configured to bend in any direction away from a longitudinal axis 601 of the delivery catheter 600 while retaining compressive and tensile stiffness. As such, the flexible delivery catheter 600 conforms to a patient's vascular structure and during placement and delivery of a medical device (e.g., prosthetic heart valve) while limiting axial deformation of the catheter 600 that can damage a patient's vascular structure.

The delivery catheter 600 includes an elongated body portion 603 and a distal tip portion 605. The elongated body portion 603 extends from the distal tip portion 605 to the proximal end 604 end the delivery catheter 600. The distal tip portion 605 extends along a length of the delivery catheter 600 from the distal end 606 to the elongated body portion 603.

The outer diameter of the catheter 600 is constant along the axial length of the elongated body portion 603 and the distal tip portion 605. In the example shown in FIG. 6, the outer diameter of the distal tip portion 605/elongated body portion 603 is about 5.4 mm. But in other examples, the outer diameter of the distal tip portion 605/elongated body portion 603 ranges between about 4.5 mm and about 6.0 mm or between about 5.0 mm and about 6.0 mm. In another example, the outer diameter of the distal tip portion 605/elongated body portion 603 is about 5.59 mm. In the example shown in FIG. 3, the inner diameter of the central lumen 602 of the delivery catheter 600 is about 4.4 mm. But in other examples, the inner diameter of the central lumen 602 of the delivery catheter 600 ranges between 3.5 mm and about 5.0 mm or between 4.0 mm and about 4.5 mm.

In the example shown in FIG. 6, the length of the distal tip portion 605, which is measured in a direction along the longitudinal axis of the delivery catheter 600, is about 285.75 mm (11.25 inches). But in some examples, the distal tip portion 605 has a length less than 508 mm (20 inches), less than 381 mm (15 inches), between 127 mm (5 inches) and 381 mm (15 inches), or between 254 mm (10 inches) and 381 mm (15 inches). In the example shown in FIG. 6, the length of the elongated body portion 603 is about 1041.4 mm (41 inches). But, in some examples, the length of the elongated body portion 603 ranges between about 762 mm (30 inches) to about 1270 mm (50 inches) or between about 889 mm (35 inches) to about 1143 mm (45 inches).

As described above, the braided layer 626 provides structural support for the catheter 600. The braided layer 626 also provides resistance to compressive deformation due to a varied density in the braid pattern. The braided layer 626 has a proximal end 630 and a distal end 632. The braided layer 626 includes a plurality of fibers 634 interwoven into a braided pattern and defines a central lumen 627, an inner surface 638, and an outer surface 640. In the example shown in FIG. 6, the braided layer 626 extends along the entire length of the catheter 600. In other examples, the braided layer 626 extends along an entire length of the distal tip portion 605. The braided layer 626 extends along at least a portion of the entire length of the elongated body portion 603 of the delivery catheter 600. In the example shown in FIG. 6, the braided layer 626 extends along the entire length of the catheter 600.

As described above, the braided layer 626 is composed of a plurality of interwoven fibers 634. Each of the fibers 634 has a curvilinear cross section, although, in other examples, the fibers 634 have a rectilinear cross section. The fibers 634 can also be flat wires to promote secure coupling of the fibers 634 in the braided pattern due to increased contact surface. The fibers 634 are composed of stainless steel, but in other examples, the fibers 634 are composed of polyester or at least one of a polymer, a metal, a composite, or any other material suitable for composing a stiffening braided layer 626 in a catheter 600. Each of the plurality of interwoven fibers 634 has a diameter of about 0.004 inches. In some examples, each of the plurality of interwoven fibers 634 has a diameter ranging between about 0.0015 inches and about 0.006 inches, between about 0.003 inches and about 0.004 inches, or between about 0.002 inches and about 0.006 inches, or between about 0.003 inches and about 0.005 inches. The interwoven fibers 634 can be oriented in a single strand braid pattern or in a double strand braid pattern where each interweave includes a plurality of parallel fibers 634 for each weave strand. The plurality of interwoven fibers 634 is composed of 16 individual fibers 634. But in other examples, the plurality of interwoven fibers 634 is composed of about 10 to about 40 individual fibers 634, or about 16 to about 32 individual fibers 634.

As described above, the braided layer 626 has a varied braid density, which allows for omnidirectional bending with respect to the central axis of the catheter 600. As the braid density increases, the braided layer 626 becomes more resistant to bending and axial compression. In the example shown in FIG. 6, the braid density of the braided layer 626 along the elongated body portion 603 is less than a braid density of the braided layer 626 along the distal tip portion 605. This allows the distal tip portion 605 to conform to the vascular structure of a patient, while the elongated body portion 603 is rigid enough to be manipulated by a physician. As described above, this increased braid density along the distal tip portion 605 provides the distal tip portion 605 with greater resistance to axial compression and bending than the elongated body portion 603. In the example shown in FIG. 6, the braid density along the entire length of the braided layer 626 varies between about 65 picks per inch and about 25 picks per inch. In other examples, the braid density along an entire length of the braided layer 626 varies between about 80 picks per inch and about 10 picks per inch. In some examples, the braid density along the distal tip portion 605 ranges between about 80 picks per inch and about 50 picks per inch or between about 60 picks per inch and about 65 picks per inch.

In some examples, the braid density along the elongated body portion 603 ranges between about 35 picks per inch and about 10 picks per inch or between about 25 picks per inch and about 20 picks per inch. The plurality of interwoven fibers 634 are woven as individual fibers 634 into the braided pattern. But in other examples, the plurality of interwoven fibers 634 are woven as fibers 634 pairs into the braided pattern, such that two braid strands are used in place of a single braid strand in the example shown in FIG. 6. In the example shown in FIG. 6, the plurality of interwoven fibers 634 extend helically about the circumference of the braided layer 626. The plurality of fibers 634 have a pitch in the distal tip portion 605 that is less than a pitch of the plurality of fibers 634 in the elongated body portion 603. A first portion of the plurality of interwoven fibers 634 extend helically about the circumference of the braided layer 626 in a first circumferential direction, and a second portion of the plurality of interwoven fibers 634 extend helically about the circumference in a second circumferential direction opposite the first circumferential direction to form the braided pattern. In some examples, the first portion of the plurality of interwoven fibers 634 extend helically about the circumference in the first direction at a first pitch, and the second portion of the plurality of interwoven fibers 634 extend helically about the circumference in the second direction at a second pitch where the first pitch is greater than the second pitch. This creates an axially narrower braid pattern that provides increased resistance to axial compression.

As described above, the catheter 600, as shown in FIG. 6 includes at least one tensile stiffening fiber 627 having a proximal end 628 and a distal end 629. The tensile stiffening fiber 627 limits tensile deformation along the axial length of the catheter 600, and limits bending of the catheter 600 is limited at a location/along the side corresponding to the circumferential location of the tensile stiffening fiber 627. For example, if the tensile stiffening fiber is located at a 90-degree position (e.g., 3 o'clock) around the circumference of the delivery catheter 600, the delivery catheter 600 will resist bending at the circumferentially 90-degree position (e.g., 3 o'clock)

The tensile stiffening fiber 627 extends along at least a portion of the entire length of the elongated body portion 603 of the delivery catheter 600. In the example shown in FIG. 6 the tensile stiffening fiber 627 extends along an entire length of the elongated body portion 603 of the delivery catheter 600. But, in other examples, a length of the at least one tensile stiffening fiber 627 corresponds to a length of the distal tip portion 605 or is greater than the length of the distal tip portion 605.

As described above with respect to catheter 300, the tensile stiffening fiber 627 is similarly coupled to the catheter 600 by the weaving/intertwining of the tensile stiffening fiber 627 into the braid pattern of the braided layer 626. The tensile stiffening fiber 627 is further secured to the braided layer 626 when the outer jacket 608 is reflowed into the braided layer 626. Like catheter 300, the tensile stiffening fiber 627 is fixedly coupled to the proximal and distal ends of the catheter 600. In the example shown in FIG. 6 the at least one tensile stiffening fiber 627 is woven into the braided layer 626 along an entire length of the braided layer 626. But, in other examples, it is contemplated that the tensile stiffening fiber 627 is woven into a portion of the length of the braided layer 626. For example, the at least one tensile stiffening fiber 627 may not be woven into a first portion of the braided layer 626 extending along the distal tip portion 605 of the delivery catheter 600 but will be woven into a second portion of the braided layer 626 extending along an elongated body portion 603. The example shown in FIGS. 6 includes Technora^{®} Kevlar. But other examples include a tensile stiffening fiber 627 composed of other Kevlar, Liquid Crystal Polymer (LCP), a polyamide type polymer, or at least one of a polymer, a metal, a composite.

The example shown in FIG. 6 includes two tensile stiffening fibers 627 spaced around the circumference of the catheter 600 by 180 degrees. But other examples include six or less, four or less, or one individual tensile stiffening fiber 627. In examples having a plurality of tensile stiffening fibers 627, the plurality of individual tensile stiffening fibers 627 can be equally spaced around the circumference of the delivery catheter 600 (e.g., every 60 degrees, 90 degrees, 120 degrees, 180 degrees), or irregularly spaced out. In examples having one individual tensile stiffening fiber 627, the at least one individual tensile stiffening fiber 627 defines the neutral axis of the delivery catheter 600 (i.e., the axis or plane along the catheter 600 at which no extension or compression occurs when the catheter is bent).

As described above, the catheter 600 includes an outer jacket 608, which forms the outermost layer of the catheter 600. The outer jacket 608 is composed of an elastic material. Like catheter 300, the elasticity of the outer jacket 608 varies (longitudinally) along a length of the outer jacket 608. In the example shown in FIG. 6, the elasticity of the outer jacket 608 increases between the proximal and distal end 611 of the outer jacket 608 such that the distal end 606 (e.g., along distal tip portion 605) of the delivery catheter 600 more easily bends to conform to the aortic geometry of a patient. Like catheter 300, the durometer of the outer jacket 608 decreases between the proximal end 604 and the distal end 606 of the catheter 600. However, as described above, the distal-most and proximal-most segments of the catheter 600 are constructed from a material having a higher durometer (and lower elasticity) than the next adjacent segment to facilitate coupling of the tensile stiffening element 627 to the proximal and distal ends of the delivery catheter 600.

The catheter 600 includes a liner 633 provided on an inner surface of the outer jacket 608, similar to liner 333 described in the aspects described above. The inner surface of the liner 633 defines an inner lumen of the delivery catheter 600. The liner 633 extends along an entire length of the inner lumen of the delivery catheter 600. In some examples, the liner 633 has a length that is greater than a length of the distal tip portion 605. In some examples, the liner 633 extends along at a portion of the entire length of the elongated body portion 603 of the delivery catheter 600 or the entire length of the elongated body portion 603 of the delivery catheter 600. The liner 633, in the example shown in FIG. 6, has a thickness of about 0.002 inches. But in other examples, the liner 633 has a thickness ranging between 0.001 inches and about 0.004 inches or between 0.0015 inches and about 0.003 inches. The liner 633 is composed of polytetrafluoroethylene (PTFE), Polyamide 12materials, polyether block amide (PEBA), fluorinated ethylene propylene (FEP), or any other polymer suitable for forming an innermost layer of a flexible delivery catheter 600.

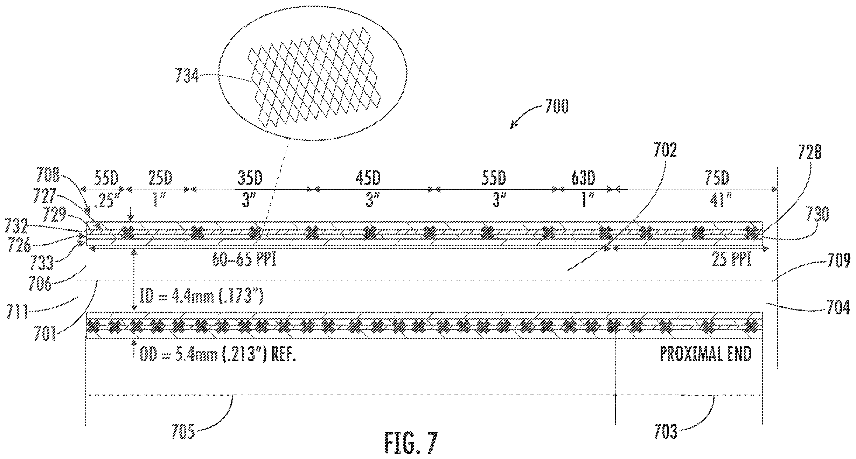

FIG. 7 shows another aspect of the flexible catheter 700, including a braided layer and tensile stiffening fiber. The catheter 700 includes similar structures and features to catheters 300, 600 described above, the differences will be discussed in more detail below, like element numbers will be used to identify like elements. The catheter 700 has an outer jacket 708, having a proximal end 704, a distal end 706, an inner surface 709, and an outer surface 710, and defining a central lumen 712 extending longitudinally between the proximal end 704 and the distal end 706. The catheter 700 includes a braided layer 726 embedded in the outer jacket 708. The braided layer 726 includes a plurality of interwoven fibers 734 disposed in a braided pattern forming an elongated tubular structure defining a central lumen 702 extending therethrough. In some examples, the braided layer 726 includes constant pitch density along the entire length of the catheter 700. The catheter 700 includes at least one tensile stiffening fiber 727 extending longitudinally along the delivery catheter 700 from the proximal end 704 to the distal end 706 of the delivery catheter 700. The at least one tensile stiffening fiber 727 limits bending of the delivery catheter 700 in a direction opposite the circumferential location of the at least one tensile stiffening fiber 727. For example, if the tensile stiffening fiber is located at a 90-degree position (e.g., 3 o'clock) around the circumference of the delivery catheter 700, the delivery catheter 700 will resist bending at the circumferentially 90-degree position (e.g., 3 o'clock). The tensile stiffening fiber 727 extends along at least a portion of the entire length of the elongated body portion 703 of the delivery catheter 700. In the example shown in FIG. 7 the least one tensile stiffening fiber 727 extends along an entire length of the elongated body portion 703 of the delivery catheter 700. But, in other examples, a length of the at least one tensile stiffening fiber 727 corresponds to a length of the distal tip portion 705 or is greater than the length of the distal tip portion 705. The tensile stiffening fiber 727 is coupled to the catheter 700 by the weaving/intertwining the tensile stiffening fiber 727 into the braid pattern of the braided layer 726. The distal ends of the tensile stiffening fiber 727 is fixedly coupled to the outer jacket 708 when the outer jacket 708 is reflowed into the braided layer 726 during manufacturing.

As shown in FIG. 7, and similar to catheters 300 and 600, the elasticity of the outer jacket 708 increases between the proximal and distal end 711 of the outer jacket 708 such that the distal end 706 of the delivery catheter 700 is configured to conform to the aortic geometry of a patient. The durometer of the outer jacket 708 decreases between the proximal end 704 and the distal end 706 of the catheter 700. As such, the outer jacket 708 includes various longitudinal segments, which are constructed from materials having varying durometer. The materials of the various longitudinal segments are reflowed together to form a single outer jacket 708. The distal end 711 of the outer jacket 708 includes a coupling segment that has lower elasticity and a higher durometer than an adjacent segment of the outer jacket 708. This segment has such a durometer to provide a material capable of suitable to securely fasten the tensile fiber 727 to. In the example shown in FIG. 7 the outer jacket 708 is composed of PEBAX@. But, in other examples, at least a portion of the outer jacket 708 comprises another polyether block amide, another elastomer, a polyamide (e.g., Vestamid^{®}), Rilsamid PA12, or any other material suitable for forming the outermost layer of a flexible catheter 700. The catheter 700 includes a liner 733. The liner 733 extends along an entire length of the inner lumen of the delivery catheter 700 and along the inner surface of the outer jacket 708. In other examples, including a braided layer 726, the catheter 700 includes a liner 733 on an inner surface of the braided layer 726.

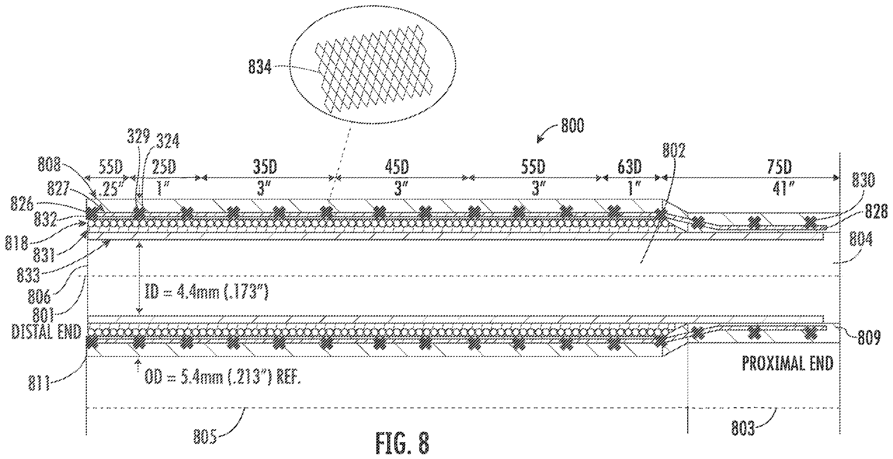

FIG. 8 shows a flexible delivery catheter 800, including a hypotube 818 rather than a coiled layer. The catheter 800 includes similar structures and features to catheters 300, 600, 700 described above, the differences will be discussed in more detail below, like element numbers will be used to identify like elements. The delivery catheter 800 includes an outer jacket 808 with a proximal end 809, a distal end 811, and a hypotube 818. The hypotube 818 has a proximal 819 end, a distal end 820, an inner surface 821, an outer surface 822. The hypotube 818 further includes a plurality of cut-outs 823, which extend between the inner surface 821 and the outer surface 822 of the hypotube 818 such that the hypotube 818 can bend omnidirectionally. The hypotube 818 is provided at the distal tip portion 805 of the delivery catheter 800.

The delivery catheter 800, in particular the distal tip portion 805, is omnidirectionally flexible and configured to bend in any direction away from a longitudinal axis 801 of the delivery catheter 800 while retaining compressive and tensile stiffness. As such, the flexible delivery catheter 800 more easily bends to conform to the patient's vascular structure during an operation while also limiting axial deformation that can damage a patient's vascular structure. The delivery catheter 800 defines a central lumen 802 extending longitudinally between the proximal end 804 and the distal end 806. Like catheters 300, 600, 700, the delivery catheter 800 includes an elongated body portion 803 and a distal tip portion 805. The distal tip portion 805 extends along a length (e.g., approx. 11.25 inches) of the delivery catheter 800 from a distal end 806 of the delivery catheter 800 toward the proximal end 804 of the delivery catheter 800, up to the elongated body portion 803 of the hypotube 818. The elongated body portion 803 extends from the distal tip portion 805 to the proximal end 804 end the delivery catheter 800 (e.g., approx. 41.0 inches). As shown in FIG. 8, the outer diameter of the distal tip portion 805 is greater than the outer diameter of the elongated body portion 803. The outer diameter/surface of the distal tip portion 805 adjacent the elongated body portion 803 includes a decreasing taper between the larger outer diameter distal tip portion 805 and the reduced diameter body portion 803.

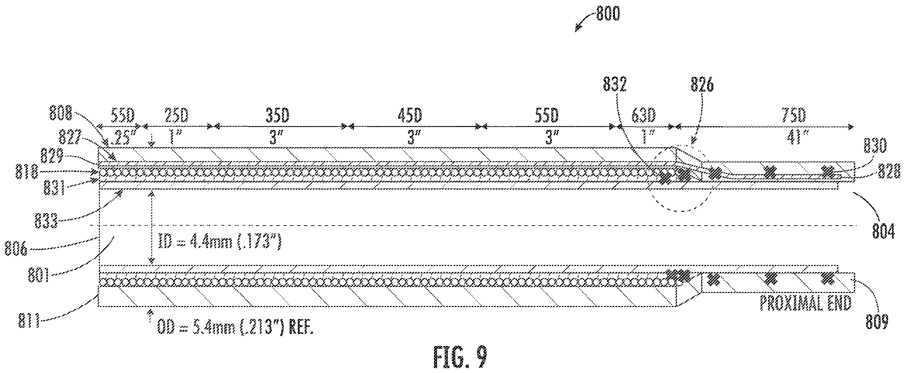

The hypotube 818 is disposed of within the distal tip portion 805. As illustrated in FIG. 8, the hypotube 818 is located on the inner lumen of the braided layer 826, where the and the braided layer 826 is disposed between the outer jacket 808 and the hypotube 818. In some examples, as illustrated in FIG. 9, the hypotube 818 is located at the distal tip portion 805, and the braided layer 826 is located along the elongated body portion 803. The braided layer 826 overlaps with a portion of the proximal end of the hypotube 818. For example, the braided layer overlaps a length of the hypotube 818 by an axial length ranging between about 0.050 inches and about 0.100" inches. As illustrated in FIG. 9, the overlapping portion of the braided layer 826 is provided on the inner diameter of the hypotube 818. It is also contemplated that the overlapping portion of the braided layer 826 can be provided on the outer diameter of the hypotube 818.

In the example shown in FIG. 8, the outer diameter of the elongated body portion 803 is about 5.4 mm. But in other examples, the outer diameter of the elongated body portion 803 ranges between about 4.5 mm and about 6.0 mm or between about 5.0 mm and about 6.0 mm. In the example shown in FIG. 8, the outer diameter of the distal tip portion 805 is about 5.59 mm. But, in other examples, the outer diameter of the distal tip portion 805 ranges between about 4.5 mm and about 6.0 mm, or between about 5.0 mm and about 6.0 mm.

Like the example catheter, 300 shown in FIG. 3, the inner diameter of the central lumen of the delivery catheter 800 is about 4.4 mm. But in other examples, the inner diameter of the central lumen of the delivery catheter 800 ranges between 3.5 mm and about 5.0 mm or between 4.0 mm and about 4.5 mm. Similar to the catheter aspects described herein, the length of the distal tip portion 805, measured in a direction along the longitudinal axis of the delivery catheter 800, is about 285.75 mm (11.25 inches). But in some examples, the distal tip portion 805 has a length less than 508 mm (20 inches), less than 381 mm (15 inches), between 127 mm (5 inches) and 381 mm (15 inches), or between 254 mm (10 inches) and 381 mm (15 inches). In the example shown in FIG. 8, the length of the elongated body portion 803 is about 1041.4 mm (41 inches). But, in some examples, the length of the elongated body portion 803 ranges between about 762 mm (30 inches) to about 1270 mm (50 inches) or between about 889 mm (35 inches) to about 1143 mm (45 inches).

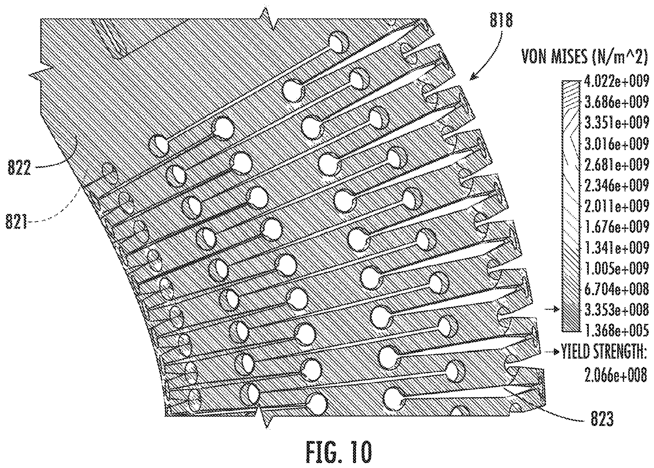

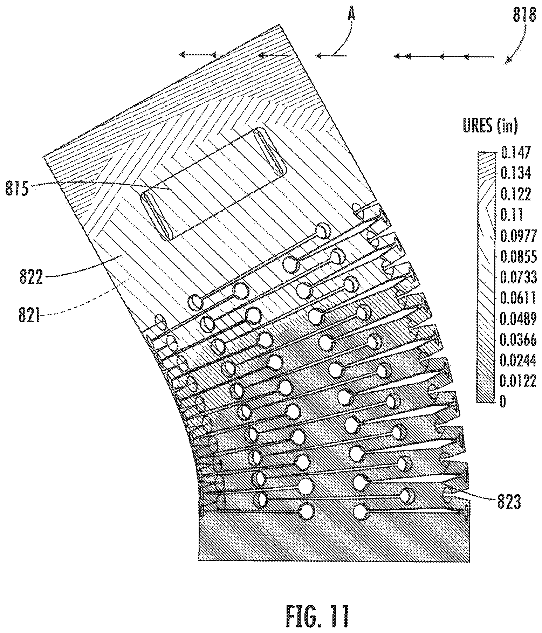

The hypotube 818, as described above, provides a layer of the catheter 800 that resists axial compression while allowing omnidirectional movement with respect to the central axis of the catheter 800. The cut-outs 823 provide bending flexibility by collapsing and expanding as the hypotube 818 bends. This expansion and contraction of the cut-outs 823 are illustrated as part of the catheter 800, as shown in FIG. 10 and FIG. 11. FIG. 10 provides a side view of the hypotube bending in response to a radial force applied at/along the distal tip portion 805. FIG. 11 illustrates a finite element analysis rendering of the hypotube of FIG. 10 in response to a stress applied in the direction of the arrow A. The hypotube 818 further provides resistance to axial compression as the cut-outs are sized and configured, having a height that provides minimal axial compression of the cutouts when an axial force is applied.

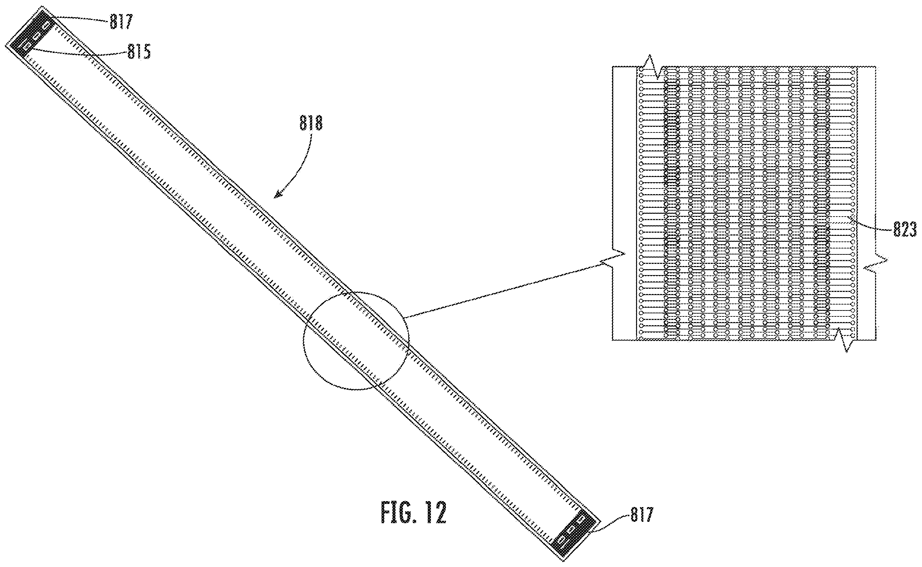

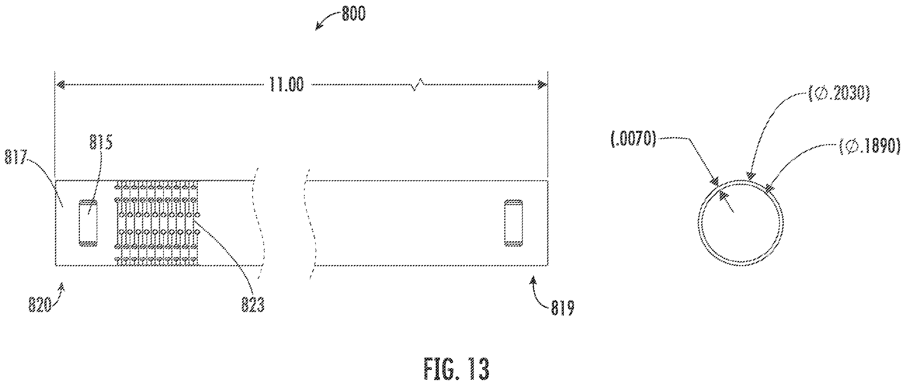

In the examples shown in FIGS 10-13, each of the plurality of cut-outs 823 are disposed in circumferential rows along the hypotube 818 (e.g., along an axial length of the hypotube 818). FIG. 11 provides a flattened side view of the hypotube 818. As shown in FIG. 11, the hypotube 818 includes rows of cut-outs 823 along the majority of the axial length of the hypotube 818. But in other examples, the hypotube 818 includes rows of cut-outs 823 along only a portion of the axial length of the hypotube 818, shown, for example, in FIG. 13 illustrating a side and end view of an example hypotube 818.

As illustrated in FIG. 11 (and FIG. 13), the hypotube 818 includes end sections 817 and both the proximal and distal ends 819, 820. The cut-outs 823 are offset from the end sections 817 of the hypotube 818 to provide structural rigidity at the proximal 819 and a distal end 820 of the hypotube 818 by providing an offset/spacing between the end of the hypotube 818 and the first (proximal or distal) row of cut-outs 823. The first row of cut-outs 823 is offset from the proximal and/or distal end 819, 820 of the hypotube 818 by about 3.81mm (0.150 inches). The end sections 817 also include anchoring windows 815. The anchoring windows 815 provide cut-outs/openings in the end sections 817 that receive reflow material (e.g., reflowed outer jacket 808 material) during manufacturing. The reflow material embeds within the anchoring windows 815, and after cooling/hardening, forms a mechanical interlock or connection between the hypotube 818 and the outer jacket 808. The hypotube 818 has a first distal-most row of cut-outs.

In some examples, each row of cutouts includes at least two cut-outs or at least four cut-outs 823. As illustrated in the enlarged portion of FIG. 11, the example hypotube 818 includes four cut-outs 823 per row. The rows of cut-outs can be distributed about the circumference of the hypotube 818 to fit the needs of the application. For example, some applications require even omnidirectional movement, while other applications require the catheter 800 to have greater resistance to bending in a first direction than in a second direction. In some examples, such as the examples shown in FIGS 10-13, each of the plurality of cut-outs 823 in the same row are distributed symmetrically about the circumference of the hypotube 818. But, in other examples, each of the plurality of cut-outs 823 in the same row are distributed asymmetrically about the circumference of the hypotube 818. As illustrated in FIG. 11, the plurality of cut-outs 823 in an adjacent row are circumferentially offset from the plurality of cut-outs 823 in an adjacent row. This promotes an even distribution of compressive resistance along the axial length of the hypotube 818.

In some examples, such as the examples shown in FIGS 10-13, each of the plurality of cut-outs 823 includes a circumferential slit and at least one semicircular hole provided at the end of the slit. The circumferential hole provides stress relief during bending and prevents unwanted damage to the hypotube 818 (e.g., by the slit breaking and/or deforming circumferentially and/or axially in the body of the hypotube 818). As illustrated in FIGS. 10-13, each of the plurality of cut-outs 823 includes a circumferential slit and a semicircular hole provided at each circumferential end of the slit.

The semicircular holes in the examples shown in FIGS. 10-13, have a radius of about 0.1524 mm (0.006 inches). In other examples, the radius of the semicircular hole ranges between about 0.1016 mm (0.004 inches) and about 0.762 mm (0.030 inches). Each of the slits has a circumferential length of about 3.3 mm (0.130 inches). But in other examples, each of the slits have a circumferential length ranging between about 0.010 mm (0.0004 inches) and about 0.025 mm (0.0010 inches). Each of the slits has an axial width/height of about 0.018 mm (0.0007 inches). But in other examples, each of the slits has an axial width/height ranging between about 0.127 mm (0.0050 inches) and about 0.254mm (0.0100 inches). The hypotube 818 has a thickness of about 0.0070 inches. But in other examples, the hypotube 818 has a thickness ranging between about 0.0050 inches and about 0.0100 inches.

The hypotube 818 has an outer diameter of about 0.2030 inches. But in some examples, the hypotube 818 has an outer diameter ranging between about 0.3000 inches and about 0.2000 inches. The hypotube 818 has an inner diameter of about 0.1890 inches. But in some examples, the hypotube 818 has an inner diameter ranging between about 0.1000 and about 0.2000 inches. In some examples, such as the example shown in FIG. 8 and FIG. 9, an overall length of the hypotube 818 is less than an overall length of the distal tip portion 805 of the delivery catheter 800. The overall length of the hypotube 818 is about 11.0 inches. But, in some examples, an overall length of the hypotube 818 ranges between 10.5 inches and about 11.5 inches. The hypotube 818 in the example shown in FIGS. 8-13 is composed of stainless steel. But in some examples, the hypotube 818 is composed of at least one of a polymer, a metal, a composite.

As described above, the catheter 800 includes a braided layer 826, which provides structural support for the catheter 800. The braided layer 826 has a proximal end 830 and a distal end 832. The braided layer 826 includes a plurality of wires 834 disposed in a braided pattern and defines a central lumen, an inner surface 838, and an outer surface 840. In the example shown in FIG. 8, the braided layer 826 extends along the entire length of catheter 800, i.e., along the elongated body portion 803 and the distal tip portion 805 of the catheter 800. But in other examples, the braided layer 826 along only a portion of the catheter. In some examples, the braided layer 826 extends along all or a portion of the entire length of the elongated body portion 803 of the delivery catheter 800. As illustrated in FIG. 9, the braided layer 826 extends along the entire length of the elongated body portion 803 and partially into the distal tip portion 805 of the catheter.

Like the catheters 300, 600, 700, the braided layer 826 is composed of a plurality of interwoven fibers 834. Each of the fibers 834 has a curvilinear cross section, although, in other examples, the fibers 834 have a rectilinear cross section. The fibers 834 are composed of stainless steel, but in other examples, the fibers 834 are composed of polyester or at least one of a polymer, a metal, or a composite, or any other material suitable for composing a stiffening braided layer 826 in a catheter 800. Each of the plurality of interwoven fibers 834 has a diameter of about 0.004 inches. In other examples, each of the plurality of interwoven fibers 834 has a diameter ranging between about 0.002 inches and about 0.006 inches or between about 0.003 inches and about 0.005 inches. The interwoven fibers 834 can be oriented in a single strand braid pattern or in a double strand braid pattern where each interweave includes a plurality of parallel fibers 834 for each weave strand. The plurality of interwoven fibers 834 is composed of 16 individual fibers 834. But in other examples, the plurality of interwoven fibers 834 is composed of about 10 to about 20 individual fibers 834. The braided layer 826 has a braid density, which allows for omnidirectional bending with respect to the central axis of the catheter 800. As the braid density increases, the braided layer 826 becomes more resistant to bending. In the example shown in FIG. 8, the braid density of the braided layer 826 is about 25 picks per inch. But in other examples, the braided layer 826 has a braid density between about 20 to about 30 picks per inch.

Similar to the catheter aspects described above, the catheter 800 shown in FIG. 8 and FIG. 9 include at least one tensile stiffening fiber 827 having a proximal end 828 and a distal end 829. As described above, the tensile stiffening fiber 827 limits tensile deformation along the axial length of the catheter 800 and limits bending of the catheter 800 in a direction opposite the circumferential location of the at least one tensile stiffening fiber 827. For example, if the tensile stiffening fiber is located at a 90-degree position (e.g., 3 o'clock) around the circumference of the delivery catheter 800, the delivery catheter 800 will resist bending at the circumferentially 90-degree position (e.g., 3 o'clock).

The tensile stiffening fiber 827 extends along at least a portion of the entire length of the elongated body portion 803 of the delivery catheter 800. In the example shown in FIG. 8 and FIG. 9, the tensile stiffening fiber 827 extends along an entire length of the elongated body portion 803 of the delivery catheter 800. But, in other examples, a length of the at least one tensile stiffening fiber 827 corresponds to a length of the distal tip portion 805 or is greater than the length of the distal tip portion 805. As described above with respect to catheter 300, the tensile stiffening fiber 827 is coupled to the other components of the catheter 800 by the weaving/intertwining the tensile stiffening fiber 827 into the braid pattern of the braided layer 826. The tensile stiffening fiber 827 is further secured to the braided layer 826 when the outer jacket 808 is reflowed into the braided layer 826. Like catheter 300, the tensile stiffening fiber 627 is fixedly coupled to the proximal and distal ends of the catheter 800. In the example shown in FIG. 8 and FIG. 9, the tensile stiffening fiber 867 is woven into the braided layer 826 along an entire length of the braided layer 826. But, in other examples, it is contemplated that the tensile stiffening fiber 827 is woven into a portion of the length of the braided layer 826. Similar to tensile stiffening fiber 327, The example shown in FIGS. 8 includes Technora^{®} Kevlar. But other examples include a tensile stiffening fiber 827 composed of other Kevlar, Liquid Crystal Polymer (LCP), a polyamide type polymer, or other polymers, metals, and various composites.

The examples shown in FIG. 8 and FIG. 9 include four tensile stiffening fibers 827 spaced around the circumference of the catheter 800 by 90 degrees. But other examples include six or less, four or less, or one individual tensile stiffening fiber 827. In examples having a plurality of tensile stiffening fibers 827, the plurality of individual tensile stiffening fibers 827 can be equally spaced around the circumference of the delivery catheter 800 (e.g., every 60 degrees, 90 degrees, 120 degrees, 180 degrees), or irregularly spaced out. In examples having one individual tensile stiffening fiber 827, the one individual tensile stiffening fiber 827 defines the neutral axis of the delivery catheter 800.

As described above, the catheter 800 includes an outer jacket 808, which forms the outermost layer of the catheter 800. The outer jacket 808 is composed of an elastic material. Like catheter 300, the elasticity of the outer jacket 808 varies (longitudinally) along a length of the outer jacket 808. In the examples shown in FIG. 8 and FIG. 9, the elasticity of the outer jacket 808 increases between the proximal and distal end 811 of the outer jacket 808 such that the distal end 806 (e.g., along the distal tip portion 805) of the delivery catheter 800 more easily bends to conform to the aortic geometry of a patient. Like catheter 300, the durometer of the outer jacket 808 decreases between the proximal end 804 and the distal end 806 of the catheter 800. However, as described above, the distal-most and proximal-most segments of the catheter 800 is constructed from a material having a higher durometer (and lower elasticity) than the next adjacent segment to facilitate coupling of the tensile stiffening element 827 to the proximal and distal ends of the delivery catheter 800.

Like catheter 300, the catheter 800 in the examples shown in FIG. 8 and FIG. 9 include a tie layer 831. The tie layer 831 is provided on an inner surface of the hypotube 818 and creates a surface for improved adhesion between the hypotube 818 and the liner 833, described below. The tie layer 831 bonds the liner 833 to the outer jacket 808. The tie layer 831 is reflowed and/or bonded with the outer jacket 808. The tie layer does not bond to the hypotube 818, instead, it encapsulates the hypotube 818 preventing material from entering between cut-outs.

As illustrated in FIG. 8 and FIG. 9, the tie layer 331 extends along the entire axial length of the inner surface of the hypotube 818. In other examples, the tie layer 831 extends along a portion of the axial length of the hypotube 818. The tie layer 831 has a thickness of about 0.003 inches, but in other examples, the tie layer 331 has a thickness ranging between about 0.002 inches and about 0.005 inches. The tie layer 331 comprises a polyether block amide material such as PEBAX@, and/or a polyamide (e.g., Vestamid^{®}), polyamide 12 (e.g., Rilsamid^{®}), or any other suitable material.

As described above, the catheter 800 includes a liner 833 provided on an inner surface of the tie layer 831. An inner surface of the liner 833 defines an inner lumen of the delivery catheter 800. The liner 833 extends along an entire length of the inner surface of the tie layer 831 or the inner lumen of the delivery catheter 800. In some examples, the liner 833 has a length that is greater than a length of the distal tip portion 805. In some examples, the liner 833 extends along at a portion of the entire length of the elongated body portion 803 of the delivery catheter 800 or the entire length of the elongated body portion 803 of the delivery catheter 800. The liner 833, in the example shown in FIG. 8, has a thickness of about 0.002 inches. But in other examples, the liner 833 has a thickness ranging between 0.001 inches and about 0.004 inches or between 0.0015 inches and about 0.003 inches. The liner 833 is composed of polytetrafluoroethylene (PTFE), PA12 materials, polyether block amide (PEBA), fluorinated ethylene propylene (FEP), or any other polymer suitable for forming an innermost layer of a flexible delivery catheter 800.

Also disclosed herein is a method of making a flexible delivery catheter 300. Similar method is contemplated for the construction of flexible delivery catheters 600, 700, 800. The method includes forming an outer jacket 308, a coil layer 318, and a braided layer 326, as described above and shown in FIG. 3 and FIG. 4. These components can be formed by molding, extrusion, or any other method suitable to form insertable medical catheter components. The coil layer 318 is disposed within the central lumen of the braid layer. The braided layer is disposed between the outer jacket 308 and the coil layer 318, and the outer jacket 308 is disposed on the outside of the coil layer 318 and the braided layer. Each of these components are coupled together and reflowed together to form a single catheter. In some examples, the coil layer 318 includes a gap/spacing between adjacent turns of the coil winding 320. In such examples, a PET layer, as described above, can be placed on either side of the coil layer 318 to prevent debris from lodging within the gap/spacing during the reflow process.

In examples wherein the outer jacket 308 includes a plurality of longitudinal segments as described above, each of the plurality of longitudinal segments are reflowed together with at least one an adjacent longitudinal segment having a differing elastic property. In examples that include tensile stiffening fibers 327, the tensile stiffening fibers 327 are coupled to the braided layer by weaving the tensile stiffening fiber 327 at least partially into the braided layer. The tensile stiffening fiber 327 is inserted into the distal tip of the outer jacket 308 and securing it thereto (e.g., via reflow and/or heat processing).

Also disclosed herein is a method of making a flexible delivery catheter 800, including a hypotube 818. The method includes forming an outer jacket 808, the hypotube 818, and a braided layer 826 as described above and shown in FIGS. 8-13. These components can be formed by molding, extrusion, or any other method suitable to form insertable medical catheter components. The hypotube 818 is disposed within the inner lumen of the braided layer 826. The braided layer 826 is disposed between the outer jacket 808 and the hypotube 818 such that the outer jacket 808 is disposed on the outside of the hypotube 818 and the braided layer 826. In another example, the hypotube is disposed within the outer jacket 808 along the distal tip portion 805 if of catheter 300, and the braided layer 826 is disposed within the outer jacket along the elongated body portion 803 of the catheter. The distal end of the braided layer can be provided either within the central lumen of the hypotube 818 and/or on the outer surface of the hypotube 818. Each of these components are coupled together and reflowed together to form a single catheter 800.

In examples wherein the outer jacket 808 comprises a plurality of longitudinal segments as described above of varying durometer, each of the plurality of longitudinal segments are reflowed together with at least one an adjacent longitudinal segment having a differing elastic property. In examples that include tensile stiffening fibers 827, the tensile stiffening fibers 827 are coupled to the braided layer 826by weaving the tensile stiffening fiber 827 at least partially into the braided layer 826. In other examples, the tensile stiffening fibers 827 are coupled to the hypotube 818 by weaving the tensile stiffening fiber 827 at least partially into the cut-outs 823 and/or the anchoring windows 815. In another example, the tensile stiffening fiber 827 is inserted into the distal tip of the outer jacket 808 and secured thereto.

Also disclosed herein is a method of making the flexible delivery catheter 700, including a braided layer 726 with a variable braid pattern. The method includes forming an outer jacket 708 and a braided layer 726 as described above and shown in FIG. 7. The braided layer is disposed within the inner lumen of the outer jacket 708. The braided layer 726 and the outer jacket 708 are coupled together and reflowed together to form a single catheter. The braided layer 726 is weaved together to form a pattern of varying density and pitch along the axial length of the hypotube.

In examples wherein the outer jacket 708 comprises a plurality of longitudinal segments as described above, each of the plurality of longitudinal segments are reflowed together with at least one an adjacent longitudinal segment having a differing elastic property. In examples that include tensile stiffening fibers 827, the tensile stiffening fibers 827 are coupled to the braided layer 826 by weaving the tensile stiffening fiber 827 at least partially into the braided pattern of the braided layer 826. The tensile stiffening fiber 827 is inserted into the distal tip of the outer jacket 808 and securing it thereto (e.g., via reflow process).

Also disclosed herein is a method of making a flexible delivery catheter 800. The method includes forming an outer jacket 808 at least one tensile stiffening fiber 827. The at least one tensile stiffening fiber 827 is embedded into the outer layer and coupled thereto.

In examples wherein the outer jacket 808 comprises a plurality of longitudinal segments as described above, each of the plurality of longitudinal segments are reflowed together with at least one an adjacent longitudinal segment having a differing elastic property. The at least one tensile stiffening fiber 827 is inserted into the distal tip of the outer jacket 808 and securing it thereto.

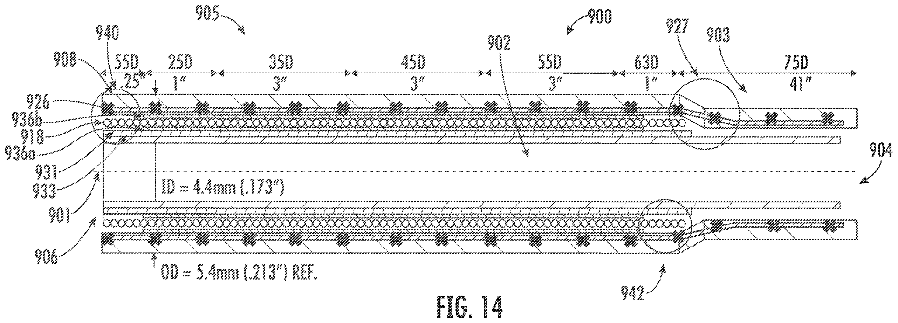

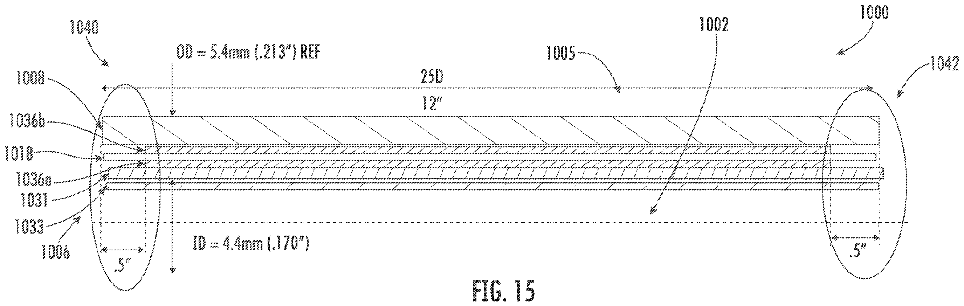

Some additional or alternative aspects are shown in FIGs. 14 and 15. The example shown in FIG. 14 includes a flexible delivery catheter 900. The delivery catheter 900 provides an omnidirectional flexible catheter that is bendable in any direction away from a longitudinal axis 901 of the delivery catheter 900 while retaining compressive and tensile stiffness. As such, and similar to all the aspects disclosed above, the flexible delivery catheter 900 conforms to a patient's vascular structure during placement and delivering a medical device (e.g., prosthetic heart valve) while limiting axial deformation of the delivery catheter 900 that can damage a patient's vascular structure.

The delivery catheter 900 defines an elongated tubular structure with a central lumen 902 extending longitudinally between the proximal end 904 and the distal end 906. The central lumen 902 (similar to the aspects disclosed above) is configured for passage of medical devices such as prosthetic heart valves therethrough.

The delivery catheter 900 further comprises an elongated body portion 903 and a distal tip portion 905. In this specific and unlimiting aspect, the distal tip portion 905 extends from the distal end of the catheter towards its proximal end for the first length of about 11.25 inches. The elongated body portion 903 extend from the most proximal portion of the distal tip portion to the proximal end of the catheter and can have a second length of about 41 inch. In this specific and unlimiting aspect, an outer diameter of the distal tip portion is larger than the outer diameter of the elongated body portion. Any ranges disclosed herein and applied to the outer diameter of the respective portion can be utilized without limitations. It is understood, however, that the aspects having a similar construction but no difference in the outer diameter between the distal tip portion and the elongated body portion is also described.

In the aspect shown in FIG. 14, the catheter comprises a tapered portion 927 that connects the distal tip portion to the elongated body portion. However, it is again understood that in the aspects where no difference in the outer diameter of the distal tip portion and the elongated body portion exist, the tapered portion 927 may not be present.

In this particular and unlimiting aspect, the delivery catheter 900 comprises and an outer jacket 908, a braided layer 926, and a stent layer 918. It is understood that in some aspects, the stent layer 918 can comprise any of the disclosed herein coil layers, or, in addition, or in the alternative, it can comprise any of the disclosed herein hypotubes. In the example shown in FIG. 14, the stent layer 918 is represented by a coil layer as described herein. This coil layer 918, similar to the aspects above, provides compressive stiffness to the catheter 900. The braided layer 926 provides structural rigidity. In contrast to aspects disclosed in FIG. 5, for example, the current aspects do not comprise the tensile stiffening fibers.

Similar to the aspects disclosed above, the coil layer 918 can have a plurality of tightly wound turns of the coil windings. In an example, coil layer 918, a gap/spacing is provided between adjacent turns of the coil winding. In another example, the coil layer 918 is tightly wound such that the adjacent turns of the coil winding contact, i.e., an outer surface of the adjacent turns of the coiled winding contact along a circumferential length of the coiled winding. This allows for some axial compression in certain applications that require partial axial compression of the catheter. In other aspects, the coil layer has substantially no gap/spacing. In yet other aspects, the coil layer can comprise any known in the art materials and any of the disclosed herein material. In some exemplary and unlimited aspects, the coil layer can comprise stainless steel. The coil layer can also comprise wired having any diameter that can provide for the desired application. In some exemplary and unlimiting aspects, the diameter of the wire used to form the coil layer can be about 0.008".

Similar to the aspects disclosed above, in this example, the coil layer 918 and the braided layer 926 are only provided at the distal tip portion 905.

The catheter 900 further comprises an inner barrier layer 936a and an outer barrier layer 936b. As it can be seen, the barrier layer 936a is disposed at at least a portion of the inner surface of the coil layer, while barrier layer 936b is disposed at at least a portion of the outer surface of the coil layer. In such aspects, the coil layer can be at least partially encapsulated within the inner and outer barrier layers. The barrier layers used in this aspect can comprise any polymers that improve the frictional properties of the coil layer and the catheter as a whole. In still further aspects, the barrier layers used in this aspect can comprise any polymers that are not melted during the reflow procedures and will stay intact and thus prevent outer materials from reflow through the coil layer. For example, and without limitation, the barrier layers used herein can comprise polyethylene terephthalate (PET) or expanded polytetrafluoroethylene (e-PTFE). The barrier layers disclosed in this aspect can have of the described above thicknesses. For example, and without limitation, it can have a thickness of 0.0005 inch. As can be further seen, the barrier layers are not present through all the lengths of the coil layer. It is understood that at least a portion of the distal tip portion does not comprise the barrier layers disclosed herein. In these exemplary aspects, the portions 940 and 942 that do not comprise the barrier layers are at the most distal end and the most proximal end of the distal tip portion. This is done to ensure that the distal and proximal ends of the coil layer are embedded into the outer layer during the reflow process and fixedly coupled within a body of the catheter.

The braided layer 926 can comprise any of the braided layers described herein. In some aspects, the braided layer 926 can comprise a plurality of fibers interwoven into a braided pattern. In the example shown in FIG. 14, the braided layer 926 extends along the entire length of the elongated body portion 903 and the distal tip portion 905 of the catheter 900. In another example, the braided layer 926 can extend along an entire length of the distal tip portion 905. The braided layer 926 can also extend along at least a portion of the entire length of the elongated body portion 903 of the delivery catheter 900. As shown herein, the braided layer 926 can extend along the entire length of the catheter 900. It is understood that the braided layer 926 can have a length that is greater than an overall length of the coil layer 918. But, in some implementations, the braided layer 926 and the coil layer 918 are about the same length along the axis of the catheter 900.

Any disclosed herein fibers can be utilized. The fibers can have any cross-section, for example, and without limitation, they can have a curvilinear cross-section or a rectilinear cross-section. The fibers can be made of any known in the art materials, for example, they can comprise at least one of a polyester, a polymer, a metal, a composite, or any other material suitable for composing a stiffening braided layer 926 in a catheter 900. Each of the plurality of interwoven fibers can have any diameter. In this example, the fibers having a diameter of about 0.004 inches are utilized. The interwoven fibers present in the braided layer 926 can be oriented in a single strand braid pattern or in a double strand braid pattern where each interweave includes a plurality of parallel fibers for each weave strand. The plurality of interwoven fibers present in the braided layer can be, for example, composed of 16 individual fibers. But in other examples, the braided layer can comprise from about 10 to about 20 individual fibers. Any of the disclosed above braid density values can be used. In this specific and unlimiting example, the braid density of 25 picks per inch can be utilized.

Similar to other aspects, the example shown in FIG. 14 also comprises a tie layer 931 and a liner 933. The tie layer 931 is provided below the inner barrier layer 936 and creates a surface for improved adhesion between the coil layer/inner barrier layer and the liner 933, which is described below. The tie layer 931 also bonds the liner 933 to the outer jacket 908 during the reflow process. As shown herein, the tie layer 931 extends along the entire axial length of the inner surface of the coil layer 918. The tie layer, however, has a length greater than a length of the barrier layers, thus allowing, while not boding to the coil layer 918, to encapsulate the coil layer 918, preventing material from entering between the coil windings. The tie layer 931 can have any thickness disclosed herein, for example, and without limitation, it can have a thickness of about 0.003 inches. The tie layer 931 can also comprise a polyether block amide material such as PEBAX@, and/or a polyamide (e.g., Vestamid^{®}), polyamide 12 (e.g., Rilsamid^{®}), or any other suitable material.

As disclosed above, the aspect shown in FIG. 14 also comprises a liner 933. The liner 933 can extend along an entire length of the inner surface of the tie layer 931 and/or the inner lumen of the delivery catheter 900, or it can extend along any portion of the catheter. Any of the disclosed above liners can be used. For example, the liner can comprise polytetrafluoroethylene (PTFE), Polyamide 12 materials, polyether block amide (PEBA), fluorinated ethylene propylene (FEP), or any other polymer suitable for forming an innermost layer of a flexible delivery catheter 900. The liner 933 in the example shown in FIG. 14 has a thickness of about 0.002 inches, but any other of the thicknesses disclosed herein can also be used.

Any of the disclosed above outer layers 908 can be utilized for this aspect.

An additional aspect is disclosed in FIG. 15. FIG. 15 illustrates the distal tip portion of the disclosed herein catheter. Any of the disclosed above elongated body portions can be used in this aspect.

The catheter 1000 comprises a distal end 1006 and a proximal end (not shown). The distal tip portion 1005 comprises an outer layer 1008, a stent layer 1018, an inner barrier layer 1036a, and an outer barrier layer 1036b. The catheter 1000, as further shown in FIG. 15, comprises a tie layer 1031 and a liner 1033. It is understood that any of the disclosed above tie layer and liners can be used in this aspect.

In this example, the stent layer can comprise a coil layer or a hypotube. Any of the disclosed above coil layers or hypotubes can be used. In this exemplary aspect, a laser-cut hypotube is utilized. The barrier layers disclosed in this aspect can comprise silicone that is spray-coated on each of the surfaces of the hypotube. It is understood that the most-distal end and the most-proximal ends of the hypotube do not comprise spray silicone coating (portions 1040 and 1042) to ensure the encapsulation of the hypotube stent layer within the tie layer and the outer jacket during the reflow process. The portions can have any length. In the example disclosed herein, the portions that do not comprise barrier layers are about 0.5" on each side of the stent layer within the distal tip portion of the catheter.

A method of deploying a prosthetic into a patient is also disclosed herein. The method includes. A delivery system is provided that includes a flexible catheter, as described above 300, 600, 700, 800. The catheter is advanced into a patient's vessel to be utilized during a medical procedure such as the insertion of a prosthetic heart valve. The prosthetic is then advanced into the patient through the catheter, and the catheter is removed.

It will be appreciated that aspects of the heart valve delivery system provide improved devices and methods for advancing a prosthetic heart valve through a patient's vasculature. In one exemplary aspect, the cooperation of components described herein allows a prosthetic valve to be advanced through the patient's vasculature and around the aortic arch in a safe manner. Accordingly, the delivery system enables advancement of a prosthetic valve around the aortic arch without requiring the introduction of an outer sheath into the aortic arch. This is an advantageous feature because the use of a sheath would increase the diameter of the delivery system, thereby complicating the delivery of the valve. In addition to providing an improved steering mechanism for navigating the aortic arch without damaging the inner wall of the aorta, it will be appreciated by those skilled in the art that the delivery system provides excellent pushability such that the physician has excellent control over the movement and location of the prosthetic valve during advancement into the native valve. This feature is particularly advantageous when traversing stenotic valve leaflets. Accordingly, aspects of the present disclosure provide an improved delivery system for advancing a prosthetic valve to the site of a native aortic valve using a steerable assembly that eliminates the need for an outer sheath in the aorta while providing sufficiently pushability to pass through narrow vasculature and/or stenotic valve leaflets. As a result, aspects of the present disclosure provide improved devices and methods for percutaneously advancing a balloon-expandable prosthetic valve to the site of a stenotic aortic valve using a retrograde approach.

## Claims

1. A flexible delivery catheter (300; 600; 700) having a proximal end and a distal end and comprising:
an outer jacket (308; 608; 708), having a proximal end, a distal end, an inner surface, and an outer surface, and defining a central lumen extending longitudinally between the proximal end and the distal end;
a coil layer (318) comprising a coil winding (320) and having a proximal end, a distal end, an inner surface, and an outer surface, wherein the coil winding (320) extends helically about a longitudinal axis (301; 601; 701) of the delivery catheter (300; 600; 700) and further defines a central lumen between the proximal end and the distal end;
a braided layer (326; 626; 726) having a proximal end and a distal end and comprising a plurality of fibers (334; 634; 734) disposed in a braided pattern and defining a central lumen having an inner surface and an outer surface,
wherein the coil layer (318) is disposed within the central lumen of the braided layer (326; 626; 726), and
wherein the braided layer (326; 626; 726) is disposed between the outer jacket (308; 608; 708) and the coil layer (318),
wherein the coil layer (318) is wound to resist axial compression and tension applied to the delivery catheter (300; 600; 700), the coil layer (318) facilitates bending of the delivery catheter (300; 600; 700) in a direction away from the longitudinal axis (301; 601; 701) of the delivery catheter (300; 600; 700),
wherein the coil layer (318) and the braided layer (326; 626; 726) are provided at a distal tip portion (305; 605; 705) of the delivery catheter (300; 600; 700), wherein the distal tip portion (305; 605; 705) has a first length extending along a length of the delivery catheter (300; 600; 700) from the distal end of the delivery catheter (300; 600; 700) toward the proximal end of the delivery catheter (300; 600; 700); and
wherein the delivery catheter (300; 600; 700) is omnidirectionally flexible and configured to bend in any direction away from the longitudinal axis (301; 601; 701) of the delivery catheter (300; 600; 700).

2. The flexible
delivery catheter (300; 600; 700) of claim 1, wherein the delivery catheter (300; 600; 700) comprises an elongated body portion (303; 603; 703) having a second length and wherein the elongated body portion (303; 603; 703) extends from the distal tip portion (305; 605; 705) to the proximal end of the delivery catheter (300; 600; 700), and wherein an outer diameter of the delivery catheter (300; 600; 700) at the distal tip portion (305; 605; 705) is greater than an outer diameter of the delivery catheter (300; 600; 700) along an elongated body portion (303; 603; 703) of the delivery catheter (300; 600; 700);
preferably wherein the outer diameter of the distal tip portion (305; 605; 705) adjacent the elongated body portion (303; 603; 703) comprises a decreasing taper between a larger outer diameter of the distal tip portion (305; 605; 705) and a reduced diameter of the elongated body portion (303; 603; 703).

3. The flexible
delivery catheter (300; 600; 700) of claim 2, wherein the first length of the distal tip portion (305; 605; 705) is less than about 520 mm, or between about 125 mm to about 385 mm, or between about 250 mm and about 385 mm, wherein the first length of the distal tip portion (305; 605; 705) is measured in a direction along the longitudinal axis (301; 601; 701) of the delivery catheter (300; 600; 700);
preferably wherein the second length of the elongated body portion (303; 603; 703) ranges between 760 mm to about 1270 mm.

4. The flexible delivery catheter (300; 600; 700) of any one of claims 1 to 3, wherein a durometer of the outer jacket (308; 608; 708) decreases between the proximal end and the distal end of the outer jacket (308; 608; 708), and wherein the durometer varies from about 25D to about 75D;
preferably wherein the outer jacket (308; 608; 708) comprises a plurality of longitudinal segments, wherein each longitudinal segment extends circumferentially, and wherein the plurality of longitudinal segments comprise materials having a varying durometer.

5. The flexible
delivery catheter (300; 600; 700) of any one of claims 1 to 4, wherein a length of the coil layer (318) is substantially identical to the first length of the distal tip portion (305; 605; 705), or wherein a length of the coil layer (318) is less than the first length of the distal tip portion (305; 605; 705); or wherein a length of the coil layer (318) is greater than the first length of the distal tip portion (305; 605; 705);
preferably wherein when the length of the coil layer (318) is greater than the first length of the distal tip portion (305; 605; 705), the coil layer (318) extends into the elongated body portion (303; 603; 703) of the delivery catheter (300; 600; 700).

6. The flexible
delivery catheter (300; 600; 700) of any one of claims 1 to 5, wherein the coil layer (318) comprises a maximum gap/spacing of about 0.25 mm between adjacent turns of the coil winding (320);
preferably wherein outer surfaces of the adjacent turns of the coil winding (320) contact along a circumferential length of the coil winding (320).

7. The flexible
delivery catheter (300; 600; 700) of any one of claims 1 to 6, wherein the coil layer (318) is coupled to the outer jacket (308; 608; 708) along the distal tip portion (305; 605; 705) of the delivery catheter (300; 600; 700);
preferably wherein the outer jacket (308; 608; 708) is reflowed together with the coil layer (318).

8. The flexible delivery catheter (300; 600; 700) of any one of claims 1 to 7, further comprising a tie layer (331) provided on the inner surface of the coil layer (318);
preferably wherein the tie layer (331) extends along at least a portion of a length of the inner surface of the coil layer (318); and/or
wherein the outer jacket (308; 608; 708) is reflowed together with the tie layer (331).

9. The flexible delivery catheter (300; 600; 700) of any one of claims 1 to 8 further comprising an outer barrier layer (336b; 936b; 1036b) provided between the outer surface of the coil layer (318) and an inner barrier layer (336a; 936a; 1036a) provided on the inner surface of the coil layer (318);
preferably wherein the outer barrier layer (336b; 936b; 1036b) is provided between the outer surface of the coil layer (318) and the outer jacket (308; 608; 708) and braided layer (326; 626; 726), wherein the inner barrier layer (336a; 936a; 1036a) is provided between the inner surface of the coil layer (318) and the tie layer (331).

10. The flexible delivery catheter (300; 600; 700) of claim 9, wherein the inner and outer barrier layers (336a, b; 936a, b; 1036a, b) are not provided at a length of the coil layer (318) extending proximally from the distal end of the coil layer (318) such that the distal end of the coil layer (318) is fixedly attached to the outer jacket (308; 608; 708);
preferably wherein the distal end of the coil layer (318) is fixedly coupled at the distal end of the delivery catheter (300; 600; 700) when the outer jacket (308; 608; 708) is bonded to the tie layer (331).

11. The flexible
delivery catheter (300; 600; 700) of any one of claims 2 to 10, wherein the braided layer (326; 626; 726) extends along the first length of the distal tip portion (305; 605; 705), or a length of the braided layer (326; 626; 726) is greater than an overall length of the coil layer (318), or wherein the braided layer (326; 626; 726) extends along at least a portion of the second length of the elongated body portion (303; 603; 703) of the delivery catheter (300; 600; 700).

12. The flexible
delivery catheter (300; 600; 700) of any one of claims 1 to 11, wherein the braided layer (326; 626; 726) does not extend to the distal tip portion (305; 605; 705) of the delivery catheter (300; 600; 700).

13. The flexible
delivery catheter (300; 600; 700) of any one of claims 1 to 12, wherein the braided layer (326; 626; 726) has a braid density varying along an axial length of the delivery catheter (300; 600; 700); and/or
wherein the braided layer (326; 626; 726) comprises of a plurality of interwoven fibers, and wherein each of the plurality of interwoven fibers have a rectilinear or curvilinear shape in cross section.

14. The flexible delivery catheter (300; 600; 700) of any one of claims 1 to 13, further comprising at least one tensile stiffening fiber (327; 827; 867) for limiting bending of the delivery catheter (300; 600; 700) in a direction opposite a circumferential location of the at least one tensile stiffening fiber (327; 827; 867);
preferably wherein a length of the at least one tensile stiffening fiber (327; 827; 867) corresponds to the first length of the distal tip portion (305; 605; 705), or wherein the length of the at least one tensile stiffening fiber (327; 827; 867) is greater than the first length of the distal tip portion (305; 605; 705), or wherein the least one tensile stiffening fiber (327; 827; 867) extends along at least a portion of the second length of the elongated body portion (303; 603; 703) of the delivery catheter (300; 600; 700), or wherein the least one tensile stiffening fiber (327; 827; 867) extends along the second length of the elongated body portion (303; 603; 703) of the delivery catheter (300; 600; 700).

15. The flexible delivery catheter (300; 600; 700) of claim 14, wherein the at least one tensile stiffening fiber (327; 827; 867) is woven into the braided layer (326; 626; 726);
preferably wherein the at least one tensile stiffening fiber (327; 827; 867) is not woven into a first portion of the braided layer (326; 626; 726) extending along the distal tip portion (305; 605; 705) of the delivery catheter (300; 600; 700), and wherein the at least one fiber is woven into a second portion of the braided layer (326; 626; 726) extending along the elongated body portion (303; 603; 703) of the delivery catheter (300; 600; 700) between the distal tip portion (305; 605; 705) and the proximal end the delivery catheter (300; 600; 700).

## Patentansprüche

1. Flexibler Zuführkatheter (300; 600; 700) mit einem proximalen Ende und einem distalen Ende und aufweisend:
einen äußeren Mantel (308; 608; 708), der ein proximales Ende, ein distales Ende, eine innere Oberfläche und eine äußere Oberfläche hat und ein zentrales Lumen definiert, das sich in Längsrichtung zwischen dem proximalen Ende und dem distalen Ende erstreckt;
eine Spulenschicht (318), die eine Spulenwicklung (320) aufweist und ein proximales Ende, ein distales Ende, eine innere Oberfläche und eine äußere Oberfläche hat, wobei sich die Spulenwicklung (320) helixförmig um eine Längsachse (301; 601; 701) des Zuführkatheters (300; 600; 700) erstreckt und ferner ein zentrales Lumen zwischen dem proximalen Ende und dem distalen Ende definiert;
eine geflochtene Schicht (326; 626; 726), die ein proximales Ende und ein distales Ende hat und eine Mehrzahl von Fasern (334; 634; 734) aufweist, die in einem geflochtenen Muster angeordnet sind und ein zentrales Lumen mit einer inneren Oberfläche und einer äußeren Oberfläche definieren,
wobei die Spulenschicht (318) innerhalb des zentralen Lumens der geflochtenen Schicht (326; 626; 726) angeordnet ist, und
wobei die geflochtene Schicht (326; 626; 726) zwischen dem äußeren Mantel (308; 608; 708) und der Spulenschicht (318) angeordnet ist;
wobei die Spulenschicht (318) gewickelt ist, um einer axialen Kompression und Spannung zu widerstehen, die auf den Zuführkatheter (300; 600; 700) ausgeübt wird, wobei die Spulenschicht (318) das Biegen des Zuführkatheters (300; 600; 700) in einer Richtung weg von der Längsachse (301; 601; 701) des Zuführkatheters (300; 600; 700) erleichtert,
wobei die Spulenschicht (318) und die geflochtene Schicht (326; 626; 726) an einem distalen Spitzenabschnitt (305; 605; 705) des Zuführkatheters (300; 600; 700) vorgesehen sind, wobei der distale Spitzenabschnitt (305; 605; 705) eine erste Länge hat, die sich entlang einer Länge des Zuführkatheters (300; 600; 700) vom distalen Ende des Zuführkatheters (300; 600; 700) hin zum proximalen Ende des Zuführkatheters (300; 600; 700) erstreckt; und
und wobei der Zuführkatheter (300; 600; 700) omnidirektional flexibel und dazu ausgebildet ist, sich in jede Richtung weg von der Längsachse (301; 601; 701) des Zuführkatheters (300; 600; 700) zu biegen.

2. Flexibler Zuführkatheter (300; 600; 700) nach Anspruch 1, wobei der Zuführkatheter (300; 600; 700) einen langgestreckten Körperabschnitt (303; 603; 703) mit einer zweiten Länge aufweist und wobei sich der langgestreckte Körperabschnitt (303; 603; 703) von dem distalen Spitzenabschnitt (305; 605; 705) zu dem proximalen Ende des Zuführkatheters (300; 600; 700) erstreckt, und wobei ein Außendurchmesser des Zuführkatheters (300; 600; 700) an dem distalen Spitzenabschnitt (305; 605; 705) größer ist als ein Außendurchmesser des Zuführkatheters (300; 600; 700) entlang eines langgestreckten Körperabschnitts (303; 603; 703) des Zuführkatheters (300; 600; 700);
wobei vorzugsweise der Außendurchmesser des distalen Spitzenabschnitts (305; 605; 705) benachbart zu dem langgestreckten Körperabschnitt (303; 603; 703) eine abnehmende Verjüngung zwischen einem größeren Außendurchmesser des distalen Spitzenabschnitts (305; 605; 705) und einem reduzierten Durchmesser des langgestreckten Körperabschnitts (303; 603; 703) aufweist.

3. Flexibler Zuführkatheter (300; 600; 700) nach Anspruch 2, wobei die erste Länge des distalen Spitzenabschnitts (305; 605; 705) weniger als etwa 520 mm, oder zwischen etwa 125 mm und etwa 385 mm, oder zwischen etwa 250 mm und etwa 385 mm beträgt, wobei die erste Länge des distalen Spitzenabschnitts (305; 605; 705) in einer Richtung entlang der Längsachse (301; 601; 701) des Zuführkatheters (300; 600; 700) gemessen wird;
wobei vorzugsweise die zweite Länge des langgestreckten Körperabschnitts (303; 603; 703) zwischen 760 mm und etwa 1270 mm liegt.

4. Flexibler Zuführkatheter (300; 600; 700) nach einem der Ansprüche 1 bis 3, wobei ein Durometer des äußeren Mantels (308; 608; 708) zwischen dem proximalen Ende und dem distalen Ende des äußeren Mantels (308; 608; 708) abnimmt und wobei das Durometer von etwa 25D bis etwa 75D variiert;
vorzugsweise wobei der äußere Mantel (308; 608; 708) eine Mehrzahl von Längssegmenten umfasst, wobei sich jedes Längssegment in Umfangsrichtung erstreckt und wobei die Mehrzahl von Längssegmenten Materialien mit einem variierenden Durometer umfasst.

5. Flexibler Zuführkatheter (300; 600; 700) nach einem der Ansprüche 1 bis 4, wobei eine Länge der Spulenschicht (318) im Wesentlichen identisch mit der ersten Länge des distalen Spitzenabschnitts (305; 605; 705) ist, oder wobei eine Länge der Spulenschicht (318) kleiner als die erste Länge des distalen Spitzenabschnitts (305; 605; 705) ist; oder wobei eine Länge der Spulenschicht (318) größer als die erste Länge des distalen Spitzenabschnitts (305; 605; 705) ist;
wobei vorzugsweise, wenn die Länge der Spulenschicht (318) größer als die erste Länge des distalen Spitzenabschnitts (305; 605; 705) ist, sich die Spulenschicht (318) in den langgestreckten Körperabschnitt (303; 603; 703) des Zuführkatheters (300; 605; 700) erstreckt.

6. Flexibler Zuführkatheter (300; 600; 700) nach einem der Ansprüche 1 bis 5, wobei die Spulenschicht (318) einen maximalen Spalt/Abstand von etwa 0,25 mm zwischen benachbarten Windungen der Spulenwicklung (320) aufweist,
wobei vorzugsweise Außenflächen benachbarter Windungen der Spulenwicklung (320) entlang einer Umfangslänge der Spulenwicklung (320) in Kontakt stehen.

7. Flexibler Zuführkatheter (300; 600; 700) nach einem der Ansprüche 1 bis 6, wobei die Spulenschicht (318) entlang des distalen Spitzenabschnitts (305; 605; 705) des Zuführkatheters (300; 600; 700) mit dem äußeren Mantel (308; 608; 708) gekoppelt ist;
wobei der äußere Mantel (308; 608; 708) vorzugsweise zusammen mit der Spulenschicht (318) umgeschmolzen wird.

8. Flexibler Zuführkatheter (300; 600; 700) nach einem der Ansprüche 1 bis 7, ferner aufweisend eine Bindeschicht (331), die auf der Innenfläche der Spulenschicht (318) vorgesehen ist;
wobei sich die Bindeschicht (331) vorzugsweise zumindest entlang eines Abschnitts einer Länge der Innenfläche der Spulenschicht (318) erstreckt; und/oder
wobei der äußere Mantel (308; 608; 708) zusammen mit der Bindeschicht (331) umgeschmolzen wird.

9. Flexibler Zuführkatheter (300; 600; 700) nach einem der Ansprüche 1 bis 8 ferner aufweisend eine äußere Sperrschicht (336b; 936b; 1036b), die vorgesehen ist zwischen der äußeren Oberfläche der Spulenschicht (318) und einer inneren Sperrschicht (336a; 936a; 1036a), die auf der Innenfläche der Spulenschicht (318) vorgesehen ist;
wobei vorzugsweise die äußere Sperrschicht (336b; 936b; 1036b) zwischen der Außenfläche der Spulenschicht (318) und dem äußeren Mantel (308; 608; 708) und der geflochtenen Schicht (326; 626; 726) vorgesehen ist, wobei die innere Sperrschicht (336a; 936a; 1036a) zwischen der Innenfläche der Spulenschicht (318) und der Spulenschicht (318) und der Bindeschicht (331) vorgesehen ist.

10. Flexibler Zuführkatheter (300; 600; 700) nach Anspruch 9, wobei die inneren und äußeren Sperrschichten (336a, b; 936a, b; 1036a, b) nicht an einer Länge der Spulenschicht (318) vorgesehen sind, die sich proximal vom distalen Ende der Spulenschicht (318) erstreckt, so dass das distale Ende der Spulenschicht (318) fest am äußeren Mantel (308; 608; 708) angebracht ist;
wobei das distale Ende der Spulenschicht (318) vorzugsweise fest mit dem distalen Ende des Zuführkatheters (300; 600; 700) gekoppelt ist, wenn der äußere Mantel (308; 608; 708) mit der Bindeschicht (331) verbunden ist.

11. Flexibler Zuführkatheter (300; 600; 700) nach einem der Ansprüche 2 bis 10, wobei sich die geflochtene Schicht (326; 626; 726) entlang der ersten Länge des distalen Spitzenabschnitts (305; 605; 705) erstreckt, oder eine Länge der geflochtenen Schicht (326; 626; 726) größer ist als eine Gesamtlänge der Spulenschicht (318), oder wobei sich die geflochtene Schicht (326; 626; 726) entlang zumindest eines Teils der zweiten Länge des langgestreckten Körperabschnitts (303; 603; 703) des Zuführkatheters (300; 600; 700) erstreckt.

12. Flexibler Zuführkatheter (300; 600; 700) nach einem der Ansprüche 1 bis 11, wobei sich die geflochtene Schicht (326; 626; 726) nicht bis zum distalen Spitzenabschnitt (305; 605; 705) des Zuführkatheters (300; 600; 700) erstreckt.

13. Flexibler Zuführkatheter (300; 600; 700) nach einem der Ansprüche 1 bis 12, wobei die geflochtene Schicht (326; 626; 726) eine Flechtdichte aufweist, die entlang einer axialen Länge des Zuführkatheters (300; 600; 700) variiert; und/oder
wobei die geflochtene Schicht (326; 626; 726) eine Mehrzahl von verflochtenen Fasern umfasst, und wobei jede der Mehrzahl von verflochtenen Fasern eine geradlinige oder gekrümmte Form im Querschnitt aufweist.

14. Flexibler Zuführkatheter (300; 600; 700) nach einem der Ansprüche 1 bis 13, ferner aufweisend zumindest eine zugfeste Versteifungsfaser (327; 827; 867) zur Begrenzung der Biegung des Zuführkatheters (300; 600; 700) in einer Richtung entgegengesetzt einer Umfangslage der zumindest einen zugfesten Versteifungsfaser (327; 827; 867);
wobei vorzugsweise eine Länge der zumindest einen zugfesten Versteifungsfaser (327; 827; 867) der ersten Länge des distalen Spitzenabschnitts (305; 605; 705) entspricht, oder wobei die Länge der zumindest einen zugfesten Versteifungsfaser (327; 827; 867) größer ist als die erste Länge des distalen Spitzenabschnitts (305; 605; 705), oder wobei sich die zumindest eine zugfeste Versteifungsfaser (327; 827; 867) entlang zumindest eines Abschnitts der zweiten Länge des langgestreckten Körperabschnitts (303; 603; 703) des Zuführkatheters (300; 600; 700) erstreckt, oder wobei die zumindest eine zugfeste Versteifungsfaser (327; 827; 867) sich entlang der zweiten Länge des Körperabschnitts (303; 603; 703) des Zuführkatheters (300; 600; 700) erstreckt.

15. Flexibler Zuführkatheter (300; 600; 700) nach Anspruch 14, wobei die zumindest eine zugfeste Versteifungsfaser (327; 827; 867) in die geflochtene Schicht (326; 626; 726) eingewoben ist;
wobei die zumindest eine zugfeste Versteifungsfaser (327; 827; 867) vorzugsweise nicht in einen ersten Abschnitt der geflochtenen Schicht (326; 626; 726) eingewoben ist, der sich entlang des distalen Spitzenabschnitts (305; 605; 705) des Zuführkatheters (300; 600; 700) erstreckt, und wobei die zumindest eine Faser in einen zweiten Abschnitt der geflochtenen Schicht (326; 626; 726) eingewoben ist, der sich entlang des länglichen Körperabschnitts (303; 603; 703) des Zuführkatheters (300; 600; 700) zwischen dem distalen Spitzenabschnitt (305; 605; 705) und dem proximalen Ende des Zuführkatheters (300; 600; 700) erstreckt.

## Revendications

1. Cathéter de pose (300 ; 600 ; 700) flexible présentant une extrémité proximale et une extrémité distale et comprenant :
une enveloppe externe (308 ; 608 ; 708), présentant une extrémité proximale, une extrémité distale, une surface interne et une surface externe et définissant une lumière centrale s'étendant longitudinalement entre l'extrémité proximale et l'extrémité distale ;
une couche de bobine (318) comprenant un enroulement de bobine (320) et présentant une extrémité proximale, une extrémité distale, une surface interne et une surface externe, l'enroulement de bobine (320) s'étendant de manière hélicoïdale autour d'un axe longitudinal (301 ; 601 ; 701) du cathéter de pose (300 ; 600 ; 700) et définissant en outre une lumière centrale entre l'extrémité proximale et l'extrémité distale ;
une couche tressée (326 ; 626 ; 726) présentant une extrémité proximale et une extrémité distale et comprenant une pluralité de fibres (334 ; 634 ; 734) disposées selon un motif tressé et définissant une lumière centrale présentant une surface interne et une surface externe,
la couche de bobine (318) étant disposée à l'intérieur de la lumière centrale de la couche tressée (326 ; 626 ; 726) et
la couche tressée (326 ; 626 ; 726) étant disposée entre l'enveloppe externe (308 ; 608 ; 708) et la couche de bobine (318),
la couche de bobine (318) étant enroulée pour résister à la compression et à la tension axiales appliquées au cathéter de pose (300 ; 600 ; 700), la couche de bobine (318) facilitant la courbure du cathéter de pose (300 ; 600 ; 700) dans un sens à l'opposé de l'axe longitudinal (301 ; 601 ; 701) du cathéter de pose (300 ; 600 ; 700),
la couche de bobine (318) et la couche tressée (326 ; 626 ; 726) étant disposées au niveau d'une partie pointe distale (305 ; 605 ; 705) du cathéter de pose (300 ; 600 ; 700), la partie pointe distale (305 ; 605 ; 705) présentant une première longueur s'étendant le long d'une longueur du cathéter de pose (300 ; 600 ; 700) à partir de l'extrémité distale du cathéter de pose (300 ; 600 ; 700) vers l'extrémité proximale du cathéter de pose (300 ; 600 ; 700) ; et
le cathéter de pose (300 ; 600 ; 700) étant omnidirectionnellement flexible et conçu pour se courber dans n'importe quel sens à l'opposé de l'axe longitudinal (301 ; 601 ; 701) du cathéter de pose (300 ; 600 ; 700).

2. Cathéter de pose (300 ; 600 ; 700) flexible selon la revendication 1, le cathéter de pose (300 ; 600 ; 700) comprenant une partie corps allongée (303 ; 603 ; 703) présentant une seconde longueur et la partie corps allongée (303 ; 603 ; 703) s'étendant à partir de la partie pointe distale (305 ; 605 ; 705) vers l'extrémité proximale du cathéter de pose (300 ; 600 ; 700) et un diamètre externe du cathéter de pose (300 ; 600 ; 700) au niveau de la partie pointe distale (305 ; 605 ; 705) étant supérieur à un diamètre externe du cathéter de pose (300 ; 600 ; 700) le long d'une partie corps allongée (303 ; 603 ; 703) du cathéter de pose (300 ; 600 ; 700) ;
de préférence, le diamètre externe de la partie pointe distale (305 ; 605 ; 705) adjacente à la partie corps allongée (303 ; 603 ; 703) comprenant une conicité décroissante entre un diamètre externe plus grand de la partie pointe distale (305 ; 605 ; 705) et un diamètre réduit de la partie corps allongée (303 ; 603 ; 703).

3. Cathéter de pose (300 ; 600 ; 700) flexible selon la revendication 2, la première longueur de la partie pointe distale (305 ; 605 ; 705) étant inférieure à environ 520 mm ou située entre environ 125 mm et environ 385 mm ou entre environ 250 mm et environ 385 mm, la première longueur de la partie pointe distale (305 ; 605 ; 705) étant mesurée dans une direction le long de l'axe longitudinal (301 ; 601 ; 701) du cathéter de pose (300 ; 600 ; 700) ;
de préférence, la seconde longueur de la partie corps allongée (303 ; 603 ; 703) étant située dans la plage de 760 mm à environ 1270 mm.

4. Cathéter de pose (300 ; 600 ; 700) flexible selon l'une quelconque des revendications 1 à 3,
un duromètre de l'enveloppe externe (308 ; 608 ; 708) diminuant entre l'extrémité proximale et l'extrémité distale de l'enveloppe externe (308 ; 608 ; 708) et le duromètre variant d'environ 25D à environ 75D ;
de préférence, l'enveloppe externe (308 ; 608 ; 708) comprenant une pluralité de segments longitudinaux, chaque segment longitudinal s'étendant de manière circonférentielle et la pluralité de segments longitudinaux comprenant des matériaux présentant un duromètre variable.

5. Cathéter de pose (300 ; 600 ; 700) flexible selon l'une quelconque des revendications 1 à 4, une longueur de la couche de bobine (318) étant sensiblement identique à la première longueur de la partie pointe distale (305 ; 605 ; 705) ou une longueur de la couche de bobine (318) étant inférieure à la première longueur de la partie pointe distale (305 ; 605 ; 705) ; ou une longueur de la couche de bobine (318) étant supérieure à la première longueur de la partie pointe distale (305 ; 605 ; 705) ;
de préférence, lorsque la longueur de la couche de bobine (318) est supérieure à la première longueur de la partie pointe distale (305 ; 605 ; 705), la couche de bobine (318) s'étendant dans la partie corps allongée (303 ; 603 ; 703) du cathéter de pose (300 ; 600 ; 700).

6. Cathéter de pose (300 ; 600 ; 700) flexible selon l'une quelconque des revendications 1 à 5, la couche de bobine (318) comprenant un interstice/écartement maximal d'environ 0,25 mm entre des spires adjacentes de l'enroulement de bobine (320) ;
de préférence, les surfaces externes des spires adjacentes de l'enroulement de bobine (320) étant en contact le long d'une longueur circonférentielle de l'enroulement de bobine (320).

7. Cathéter de pose (300 ; 600 ; 700) flexible selon l'une quelconque des revendications 1 à 6, la couche de bobine (318) étant accouplée à l'enveloppe externe (308 ; 608 ; 708) le long de la partie pointe distale (305 ; 605 ; 705) du cathéter de pose (300 ; 600 ; 700) ;
de préférence, l'enveloppe externe (308 ; 608 ; 708) étant refondue conjointement avec la couche de bobine (318).

8. Cathéter de pose (300 ; 600 ; 700) flexible selon l'une quelconque des revendications 1 à 7, comprenant en outre une couche de liaison (331) disposée sur la surface interne de la couche de bobine (318) ;
de préférence, la couche de liaison (331) s'étendant le long d'au moins une partie d'une longueur de la surface interne de la couche de bobine (318) ; et/ou
l'enveloppe externe (308 ; 608 ; 708) étant refondue conjointement avec la couche de liaison (331).

9. Cathéter de pose (300 ; 600 ; 700) flexible selon l'une quelconque des revendications 1 à 8, comprenant en outre une couche barrière externe (336b ; 936b ; 1036b) disposée entre la surface externe de la couche de bobine (318) et une couche barrière interne (336a ; 936a ; 1036a) disposée sur la surface interne de la couche de bobine (318) ;
de préférence, la couche barrière externe (336b ; 936b ; 1036b) étant disposée entre la surface externe de la couche de bobine (318) et l'enveloppe externe (308 ; 608 ; 708) et la couche tressée (326 ; 626 ; 726), la couche barrière interne (336a ; 936a ; 1036a) étant disposée entre la surface interne de la couche de bobine (318) et la couche de liaison (331).

10. Cathéter de pose (300 ; 600 ; 700) flexible selon la revendication 9, les couches barrière interne et externe (336a, b ; 936a, b ; 1036a, b) n'étant pas disposées à une longueur de la couche de bobine (318) s'étendant de manière proximale à partir de l'extrémité distale de la couche de bobine (318) de telle sorte que l'extrémité distale de la couche de bobine (318) est attachée de manière fixe à l'enveloppe externe (308 ; 608 ; 708) ;
de préférence, l'extrémité distale de la couche de bobine (318) étant accouplée de manière fixe au niveau de l'extrémité distale du cathéter de pose (300 ; 600 ; 700) lorsque l'enveloppe externe (308 ; 608 ; 708) est liée à la couche de liaison (331).

11. Cathéter de pose (300 ; 600 ; 700) flexible selon l'une quelconque des revendications 2 à 10, la couche tressée (326 ; 626 ; 726) s'étendant le long de la première longueur de la partie pointe distale (305 ; 605 ; 705) ou une longueur de la couche tressée (326 ; 626 ; 726) étant supérieure à une longueur totale de la couche de bobine (318) ou la couche tressée (326 ; 626 ; 726) s'étendant le long d'au moins une partie de la seconde longueur de la partie corps allongée (303 ; 603 ; 703) du cathéter de pose (300 ; 600 ; 700).

12. Cathéter de pose (300 ; 600 ; 700) flexible selon l'une quelconque des revendications 1 à 11, la couche tressée (326 ; 626 ; 726) ne s'étendant pas jusqu'à la partie pointe distale (305 ; 605 ; 705) du cathéter de pose (300 ; 600 ; 700).

13. Cathéter de pose (300 ; 600 ; 700) flexible selon l'une quelconque des revendications 1 à 12, la couche tressée (326 ; 626 ; 726) présentant une densité de tresse variant le long d'une longueur axiale du cathéter de pose (300 ; 600 ; 700) ; et/ou
la couche tressée (326 ; 626 ; 726) comprenant une pluralité de fibres entrelacées et chacune de la pluralité de fibres entrelacées présentant une forme rectiligne ou curviligne en coupe transversale.

14. Cathéter de pose (300 ; 600 ; 700) flexible selon l'une quelconque des revendications 1 à 13, comprenant en outre au moins une fibre de raidissement à la traction (327 ; 827 ; 867) destinée à limiter la courbure du cathéter de pose (300 ; 600 ; 700) dans un sens opposé à un emplacement circonférentiel de ladite au moins une fibre de raidissement à la traction (327 ; 827 ; 867) ;
de préférence, une longueur de ladite au moins une fibre de raidissement à la traction (327 ; 827 ; 867) correspondant à la première longueur de la partie pointe distale (305 ; 605 ; 705) ou la longueur de ladite au moins une fibre de raidissement à la traction (327 ; 827 ; 867) étant supérieure à la première longueur de la partie pointe distale (305 ; 605 ; 705) ou ladite au moins une fibre de raidissement à la traction (327 ; 827 ; 867) s'étendant le long d'au moins une partie de la seconde longueur de la partie corps allongée (303 ; 603 ; 703) du cathéter de pose (300 ; 600 ; 700) ou ladite au moins une fibre de raidissement à la traction (327 ; 827 ; 867) s'étendant le long de la seconde longueur de la partie corps allongée (303 ; 603 ; 703) du cathéter de pose (300 ; 600 ; 700).

15. Cathéter de pose (300 ; 600 ; 700) flexible selon la revendication 14, ladite au moins une fibre de raidissement à la traction (327 ; 827 ; 867) étant tissée dans la couche tressée (326 ; 626 ; 726) ;
de préférence, ladite au moins une fibre de raidissement à la traction (327 ; 827 ; 867) n'étant pas tissée dans une première partie de la couche tressée (326 ; 626 ; 726) s'étendant le long de la partie pointe distale (305 ; 605 ; 705) du cathéter de pose (300 ; 600 ; 700) et ladite au moins une fibre étant tissée dans une seconde partie de la couche tressée (326 ; 626 ; 726) s'étendant le long de la partie corps allongée (303 ; 603 ; 703) du cathéter de pose (300 ; 600 ; 700) entre la partie pointe distale (305 ; 605 ; 705) et l'extrémité proximale du cathéter de pose (300 ; 600 ; 700).
